# EUROPEAN PATENT APPLICATION

(11) **EP 4 644 367 A1**
(43) Date of publication of application: **05.11.2025**
(21) Application number: 23912013.2
(22) Date of filing: 25.12.2023
(51) Int. Cl.: C07C 319/02, C07C 319/22, C07C 321/02, C07C 321/14, C08G 18/38

(54) **METHOD FOR PRODUCING POLYTHIOL COMPOSITION, METHOD FOR PRODUCING POLYMERIZABLE COMPOSITION, AND METHOD FOR PRODUCING RESIN**

(30) Priority: 27.12.2022 JP 2022209573
(71) Applicant: HOYA Lens Thailand Ltd., Thanyaburi, Pathumthani 12130 (TH)
(72) Inventor: TANIZAWA, Hideya, Tokyo 160-8347 (JP); KAWAKAMI, Hironori, Tokyo 160-8347 (JP)
(74) Representative: Kraus & Lederer PartGmbB
(86) International application number: PCT/JP2023/046290
(87) International publication number: WO 2024/143233

(57) **Abstract**

A method for producing a polythiol composition including a reaction step of reacting a thiourethane resin and a nitrogen-containing compound that is at least one selected from the group consisting of a quaternary ammonium salt and an amine compound in the presence of water to generate a polythiol composition.

## Description

### Technical Field

The present disclosure relates to a method for producing a polythiol composition, a method for producing a polymerizable composition, and a method for producing a resin.

### Background Art

Plastic lenses, which are lenses containing a resin, are lightweight and less likely to break compared with inorganic lenses and can be dyed and have thus become widespread rapidly in applications such as spectacle lenses and camera lenses.

For example, various studies have been thus far made regarding lenses including a thiourethane resin (for example, refer to PTL 1 to PTL 3).

As raw materials for producing the thiourethane resin (hereinafter, also referred to as "thiourethane resin raw materials"), for example, polythiol compositions such as 4,8-dimercaptomethyl-1,11-dimercapto-3,6,9-trithiaundecane and polyisocyanate compounds such as m-xylylene diisocyanate (XDI) are used. The polyisocyanate compounds are made from, for example, a polyamine compound. The polyamine compound, which is a raw material of the polyisocyanate compounds, also corresponds to the thiourethane resin raw material (that is, a raw material for producing the thiourethane resin).

A lens containing a thiourethane resin (for example, a spectacle lens) is manufactured by cutting a molded body containing a thiourethane resin. As a result, in the lens manufacturing process, a large amount of chips containing a thiourethane resin may be generated as waste. In addition, in the step of manufacturing a molded body containing a thiourethane resin, a defective molded body or a defective processed body may be generated. Such waste is usually not effectively used (that is, recycled), and is simply incinerated or buried.

Therefore, from the viewpoint of the effective use of materials, a technique for producing a polythiol composition using chips containing a thiourethane resin, which is waste, or a defective molded or processed product as a starting raw material has been developed (for example, refer to PTL 4).

Here, PTL 4 discloses that a polythiol composition is obtained by a reaction in which an amine compound as a base and an organic solvent that is poorly miscible with water (high-boiling point alcohol, toluene) are used.

### Citation List

### Patent Literature

Patent Literature 1: Japanese Patent Application Publication No. S63-46213
Patent Literature 2: Japanese Patent Application Publication No. H02-270859
Patent Literature 3: Japanese Patent Application Publication No. H07-252207
Patent Literature 4: WO 2021/157701

### Summary of Invention

### Technical Problem

However, it was not possible to efficiently perform the reaction at the time of producing the polythiol composition and to obtain the polythiol composition within a short period of time. Therefore, from the viewpoint of reducing energy consumption and suppressing the generation of a by-product, there has been a strong desire for development of a reaction system capable of producing a polythiol composition in a short reaction time using a thiourethane resin as a starting material.

Under such circumstances, one aspect of the present disclosure aims to provide a method for producing a polythiol composition enabling a polythiol composition to be produced in a short reaction time using a thiourethane resin as a starting raw material, a method for producing a polymerizable composition using the method for producing the polythiol composition, and a method for producing a resin using the method for producing the polymerizable composition.

### Solution to Problem

An embodiment of the present disclosure relates to the following [1] to [8].
[1] A method for producing a polythiol composition, including: a reaction step of reacting a thiourethane resin and a nitrogen-containing compound that is at least one selected from the group consisting of a quaternary ammonium salt and an amine compound in the presence of water to generate a polythiol composition.
[2] The method for producing the polythiol composition according to [1], wherein a content of water in a reaction system of the reaction step is 2 to 50 mass%.
[3] The method for producing the polythiol composition according to [1] or [2], wherein the quaternary ammonium salt comprises a quaternary ammonium cation and a counter anion, and the counter anion is a hydroxide ion.
[4] The method for producing the polythiol composition according to any one of [1] to [3], wherein the amine compound is at least one selected from the group consisting of a primary amine, a secondary amine, and a tertiary amine.
[5] The method for producing the polythiol composition according to any one of [1] to [4], wherein the reaction system in the reaction step further contains an alcohol.
[6] The method for producing a polythiol composition according to [5], wherein the alcohol contains one or more alcohols miscible with water.
[7] A method for producing a polymerizable composition, including: a step of producing a polythiol composition by the method for producing a polythiol composition according to any one of [1] to [6]; and a step of mixing a polythiol composition containing the produced polythiol composition and a polyisocyanate compound to obtain a polymerizable composition containing the polythiol composition and the polyisocyanate compound.
[8] A method for producing a resin, including: a step of producing a polymerizable composition by the method for producing a polymerizable composition according to [7]; and a step of curing the polymerizable composition to obtain a resin.

### Advantageous Effects of Invention

According to one aspect of the present disclosure, there can be provided a method for producing a polythiol composition enabling a polythiol composition to be produced in a short reaction time using a thiourethane resin as a starting raw material, a method for producing a polymerizable composition using the method for producing the polythiol composition, and a method for producing a resin using the method for producing the polymerizable composition.

### Description of Embodiments

Hereinafter, one example of an embodiment of the present disclosure will be described. However, the following embodiment is illustrative for embodying the technical concept of the present disclosure, and the present disclosure is not limited to the following description.

An aspect obtained by optionally selecting or optionally combining matters described in the present specification is also included in the present disclosure.

In the present specification, a preferable regulation can be optionally selected, and a combination of preferable regulations can be said to be more preferable.

In the present specification, expression "XX to YY" means "XX or more and YY or less."

In the present specification, the lower limit values and the upper limit values stated stepwise regarding preferable numerical ranges (for example, the ranges of contents and the like) can be each independently combined together. For example, from the description "preferably 10 to 90 and more preferably 30 to 60," "preferable lower limit value (10)" and "more preferable upper limit value (60)" can be combined to form "10 to 60."

In the present specification, in a case where a plurality of substances corresponding to each component are present in a composition, unless particularly otherwise described, the amount of each component that is contained in the composition means the total amount of the plurality of substances present in the composition.

In the present specification, the term "step" includes not only an independent step but also a step that cannot be clearly distinguished from other steps as long as the desired purpose of the step is achieved.

In the present specification, the term "reaction system" means "a reaction system of a reaction step in a method for producing a polythiol composition."

In the reaction system in the reaction step, in addition to essential components including a thiourethane resin, a nitrogen-containing compound, and water, an optional component such as an alcohol or a reaction solvent is also contained. Accordingly, the content (mass%) in the reaction system means the content (mass%) in a case where the total content of the essential components and the optional component in the reaction system is set to 100 mass%.

In the present specification, in the case of a reaction system containing a compound corresponding to both an alcohol and an amine compound, such as monoethanolamine, instead of counting both the alcohol and the amine compound, only one of the alcohol and the amine compound is counted in calculating "the total content of the essential components and the optional component in the reaction system." Here, "the content (mass%) of the alcohol in the reaction system" is a value obtained by dividing "the mass of the compound corresponding to both the alcohol and the amine compound" by "the total content of the essential components and the optional component in the reaction system" calculated by counting only one of the alcohol and the amine compound, "the content (mass%) of the amine compound in the reaction system" is a value obtained by dividing "the mass of the compound corresponding to both the alcohol and the amine compound" by "the total content of the essential components and the optional component in the reaction system" calculated by counting only one of the alcohol and the amine compound, and "the content (mass%) of the alcohol in the reaction system" and "the content (mass%) of the amine compound in the reaction system" are the same values.

In the present specification, the reaction system (composition) "including as a main component" a certain component (hereinafter, referred to as "component X") means that the content of component X (the total content of two or more compounds in a case where the component X is composed of two or more compounds) is 50 mass% or more relative to the total amount of the reaction system (composition).

The content of the component X, which is a main component, is preferably 60 mass% or more, more preferably 70 mass% or more, and particularly preferably 80 mass% or more relative to the total amount of the reaction system (composition).

In the present specification, "high boiling point" in "high-boiling point alcohol" means 140°C or higher.

In the present specification, the definitions and illustrations of R³, R⁴, R⁵, R⁶, R¹¹, and R¹² are the same as the definition and illustration of R in quaternary ammonium.

### [Method for Producing Polythiol Composition]

A method for producing a polythiol composition according to the embodiment of the present disclosure includes a reaction step of reacting a thiourethane resin and a nitrogen-containing compound that is at least one selected from the group consisting of a quaternary ammonium salt and an amine compound in the presence of water to generate a polythiol composition.

In the method for producing a polythiol composition according to the embodiment of the present disclosure, since a thiourethane resin and a nitrogen-containing compound that is at least one selected from the group consisting of a quaternary ammonium salt and an amine compound are reacted with each other in the presence of water, it is possible to produce a polythiol composition in a short reaction time using the thiourethane resin as a starting raw material.

In a case where the reaction system further contains an alcohol, alcoholysis in which the thiourethane resin is further degraded by the alcohol occurs in the reaction step, whereby a polythiol composition, which is the target substance, is generated by this alcoholysis.

In a case where the reaction system further contains an alcohol, it is considered that in the reaction step, the alcohol functions as a degradation agent in the alcoholysis, and the nitrogen-containing compound functions as a degradation aid in the alcoholysis.

In a case where the reaction system contains no alcohols, it is considered that a counter anion or the amine compound itself in the quaternary ammonium salt as the nitrogen-containing compound undergoes a nucleophilic reaction to carbon of a carbonyl group of the thiourethane resin (refer to a reaction formula (X), a reaction formula (6), and a reaction formula (7) shown below).

The method for producing a polythiol composition according to the embodiment of the present disclosure includes at least the reaction step, and if necessary, includes a separation step; and other steps such as a classification step, a sieving step, a washing step, and a crushing (pulverizing) step.

Hereinafter, each step that may be included in the method for producing a polythiol composition will be described.

### [[Reaction Step]]

The reaction step is a step of reacting a thiourethane resin, a nitrogen-containing compound, and, if necessary, an optional component such as an alcohol in the presence of water to generate a polythiol composition.

### <Thiourethane Resin>

The thiourethane resin is a starting raw material in the present step and the method for producing a polythiol composition.

The thiourethane resin is not particularly limited, and examples thereof include thiourethane resins described in known publications such as Japanese Patent Application Publication No. S63-46213, Japanese Patent Application Publication No. H02-270859, Japanese Patent Application Publication No. H07-252207, WO 2008/047626, and the like.

Thiourethane resins are usually produced from a polyisocyanate compound and a polythiol composition as raw materials as a reaction product thereof.

Examples of the thiourethane resins include thiourethane resins obtained from MR-6, MR-7, MR-8, MR-8Plus, MR-60, MR-10, and MR-20, which are high-refractive index lens materials, (all manufactured by Mitsui Chemicals Inc.); EYAS 1.60 (manufactured by HOYA Corporation); and the like.

The thiourethane resin is preferably a resin collected in at least one of a manufacturing process of a spectacle lens, a manufacturing process of spectacles, and a disposal process of spectacles. According to this aspect, recycling of the thiourethane resin, which is a spectacle lens material, is achieved.

Here, the manufacturing process of a spectacle lens means a process of producing a resin by mixing monomers, which are resin raw materials, and performing cast polymerization, and/or a process of obtaining a spectacle lens by cutting a resin molded body, the manufacturing process of spectacles means a process of manufacturing spectacles by combining a spectacle lens and another member such as a spectacle frame, and the disposal process of spectacles means a process of disposing of spectacles that have been manufactured but become unnecessary, used spectacles, and the like.

In any process, a thiourethane resin, which is a material of a spectacle lens, can be generated as waste.

It is preferable to use a thiourethane resin generated in at least one of these processes as a starting material and reacting this thiourethane resin, a nitrogen-containing compound, and if necessary, an optional component such as an alcohol in the presence of water to obtain a polythiol composition, which is a degraded product of the thiourethane resin.

As described above, in the method for producing a polythiol composition of the present disclosure, a used thiourethane resin is used to produce a polythiol composition, whereby it becomes possible to reduce the amount of the thiourethane resin to be disposed of by incineration and, as a result, to reduce the generation of greenhouse gases such as carbon dioxide and air pollutants such as a sulfur oxide and a nitrogen oxide. In addition, since thiourea is not used for the production of a polythiol composition, no waste water containing thiourea is generated, and the production method is environmentally-friendly.

This will be described using a specific example. In a case where 1 kg of the thiourethane resin is disposed of by incineration, the contents of carbon atoms, nitrogen atoms, and sulfur atoms in the thiourethane resin becomes 48.5 mass%, 7.6 mass%, and 30.2 mass%, respectively. In a case where this thiourethane resin is combusted, various types of oxides of carbon atoms, nitrogen atoms, and sulfur atoms are generated as gases by the combustion method; however, when the products are carbon dioxide, nitrogen monoxide, and sulfur dioxide, 1.78 kg of carbon dioxide, 0.16 kg of nitrogen monoxide, and 0.6 kg of sulfur dioxide are generated when 1 kg of the thiourethane resin is disposed of.

In the method for producing a polythiol composition of the present disclosure, it becomes possible to reduce the generation of these carbon dioxide, nitrogen monoxide, and sulfur dioxide.

The starting raw material preferably contains chips containing the thiourethane resin.

In the step of generating a polythiol composition in this aspect, chips containing the thiourethane resin, a nitrogen-containing compound, and if necessary, an optional component such as an alcohol are brought into contact with one another in the presence of water, thereby reacting the thiourethane resin, the nitrogen-containing compound, and the optional component such as an alcohol in the presence of the water.

In this aspect, the reactivity of the nitrogen-containing compound, the thiourethane resin in the starting raw material, the optional component such as an alcohol, and water is superior, and a polythiol composition can be thus generated more effectively.

### (Powder Containing Thiourethane Resin)

In the reaction step, it is preferable that a powder containing the thiourethane resin (hereinafter, also referred to as "thiourethane resin powder"), a nitrogen-containing compound, and an optional component such as an alcohol are brought into contact with one another in the presence of water, thereby reacting the thiourethane resin in the powder, the nitrogen-containing compound, the optional component such as an alcohol, and water with one another. This makes it possible to further improve the reaction efficiency of the thiourethane resin, the nitrogen-containing compound, the optional component such as an alcohol, and water.

A method for bringing the thiourethane resin, the nitrogen-containing compound, the optional component such as an alcohol, and water into contact with one another is not particularly limited, and examples thereof include methods in which the thiourethane resin powder, the nitrogen-containing compound, and water (and, if necessary, an alcohol, a reaction solvent, or the like) are fed into a reaction vessel and stirred. In this example, the order of feeding the thiourethane resin powder, the nitrogen-containing compound, and water (and, if necessary, the alcohol, the reaction solvent, or the like) into the reaction vessel is not particularly limited.

The thiourethane resin powder is not particularly limited, but is preferably chips of a molded body containing the thiourethane resin (including the concept of a polishing powder, which will be also true below) and/or the sieved chips (that is, the chips that have been passed through a sieve).

The chips of the molded body containing the thiourethane resin are generated, for example, when the molded body containing the thiourethane resin is cut to manufacture an optical material (for example, a lens).

In addition, the thiourethane resin powder may be an agglomerated powder obtained by crushing and/or pulverizing the molded body containing the thiourethane resin.

The content of the thiourethane resin in the reaction system of the reaction step is not particularly limited, but is preferably 2 to 50 mass%, more preferably 4 to 30 mass%, and particularly preferably 6 to 20 mass% from the viewpoint of further improving the reactivity of the thiourethane resin.

### (Polyisocyanate Compound as Raw Material of Thiourethane Resin)

The polyisocyanate compound as a raw material of the thiourethane resin may be only one kind or two or more kinds.

The polyisocyanate compound as a raw material of the thiourethane resin preferably contains a polyisocyanate compound containing two or more isocyanate groups.

Examples of the polyisocyanate compound as a raw material of the thiourethane resin include known polyisocyanate compounds described in the above-described known publications (that is, Japanese Patent Application Publication No. S63-46213, Japanese Patent Application Publication No. H02-270859, Japanese Patent Application Publication No. H07-252207, WO 2008/047626, and the like).

### (Polythiol Composition as Raw material for Thiourethane Resin)

The polythiol composition as a raw material for the thiourethane resin may be only one kind of polythiol compound or may be two or more kinds of polythiol compounds.

The polythiol composition as a raw material for the thiourethane resin needs to contain a polythiol compound having two or more thiol groups (that is, mercapto groups), and there are no additional particular limitations.

Examples of the polythiol composition as a raw material for the thiourethane resin include known polythiol compositions described in the above-described known publications (that is, Japanese Patent Application Publication No. S63-46213, Japanese Patent Application Publication No. H02-270859, Japanese Patent Application Publication No. H07-252207, WO 2008/047626, and the like).

The thiourethane resin may contain at least one different component other than a polymer of the polyisocyanate compound and the polythiol composition.

Regarding the different component that can be contained in the thiourethane resin, a component that can be contained in a polymerizable composition described below can be referred to as appropriate.

### <Nitrogen-Containing Compound>

The nitrogen-containing compound is at least one selected from the group consisting of a quaternary ammonium salt and an amine compound. That is, the nitrogen-containing compound may be any of a quaternary ammonium salt alone, an amine compound alone, and a of a quaternary ammonium salt and an amine compound.

The content of the nitrogen-containing compound in the reaction system of the reaction step is not particularly limited, but is preferably 1 to 40 mass%, more preferably 2 to 30 mass% and particularly preferably 3 to 20 mass% from the viewpoint of further improving the reactivity with the thiourethane resin.

### (Amount of Nitrogen-Containing Compound Charged)

In the reaction step, the mass ratio of the nitrogen-containing compound charged to the thiourethane resin (that is, the charge mass ratio [nitrogen-containing compound/thiourethane resin]) can be adjusted as appropriate, but is preferably, 0.1 to 10, more preferably, 0.2 to 9, and particularly preferably 0.4 to 6.

In a case where the charge mass ratio [nitrogen-containing compound/thiourethane resin] is 0.1 or more, the generation of the polythiol composition is further promoted.

In a case where the charge mass ratio [nitrogen-containing compound/thiourethane resin] is 10 or less, the remaining of the nitrogen-containing compound in a reaction mixture can be further suppressed.

In the reaction step, the millimolar number of the nitrogen-containing compound charged relative to 1 g of the thiourethane resin is preferably 1.0 to 100.0 mmol/g, more preferably 2.0 to 50.0 mmol/g, and particularly preferably 3.0 to 25.0 mmol/g.

In the reaction step, the charge equivalent of the nitrogen-containing compound relative to the thiourethane resin (charge equivalent [nitrogen-containing compound/thiourethane resin]) is preferably 1.0 to 10.0, more preferably 1.0 to 8.0, and particularly preferably 1.0 to 6.0.

In a case where the charge equivalent [nitrogen-containing compound/thiourethane resin] is 1.0 or more, the generation of the polythiol composition is further promoted.

In a case where the charge equivalent [nitrogen-containing compound/thiourethane resin] is 10.0 or less, the remaining of the nitrogen-containing compound in the reaction mixture can be further suppressed.

### <<Quaternary Ammonium Salt>>

The quaternary ammonium salt is not particularly limited as long as the quaternary ammonium salt is composed of a quaternary ammonium cation and a counter anion, and suitable examples thereof suitably include tetramethylammonium hydroxide (TMAH), tetraethylammonium hydroxide (TEAH), tetrabutylammonium hydroxide (TBAOH), benzyltrimethylammonium hydroxide, tetrahexylammonium hydroxide, 2-hydroxyethyltrimethylammonium hydroxide, hexadecyltrimethylammonium hydroxide, N,N,N-tris(polyoxyethylene)-N-methylammonium hydroxide, trimethylphenylammonium hydroxide, 3-(trifluoromethyl)phenyltrimethylammonium hydroxide, tetrabutylammonium fluoride (TBAF), tetrabutylammonium chloride (TBACl), tetrabutylammonium bromide (TBAB), tetrabutylammonium iodide (TBAI), benzyltriethylammonium hydroxide, hexadecyltrimethylammonium hydroxide, benzethonium hydroxide, benzalkonium hydroxides, cetylpyridinium hydroxide, and the like.

In a case where the reaction system contains an alcohol as the optional component, as shown in the following reaction formula (1), "quaternary ammonium salt (quaternary ammonium cation NR₄⁺ and counter anion X⁻)" and "alcohol R³OH" on the left side are reacted with each other, whereby "NR₄⁺" and "R³O⁻" on the right side are generated. As shown in the following reaction formula (2), it is assumed that the generated "R³O⁻" promotes the degradation of the thiourethane resin. Here, when "R³O⁻" is excessively generated, it is assumed that a side reaction progresses. Therefore, in the present invention, the excessive generation of "R³O⁻" is prevented by adding water to the alcohol as the optional component to adjust the alcohol concentration, whereby a side reaction is suppressed, and a polythiol composition can be produced in a short reaction time.

In a case where the reaction system does not contain any alcohol as the optional component, as shown in the following reaction formula (X), it is assumed that the generated "X⁻" promotes the degradation of the thiourethane resin.

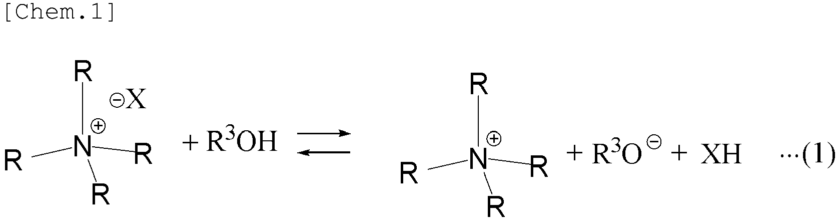

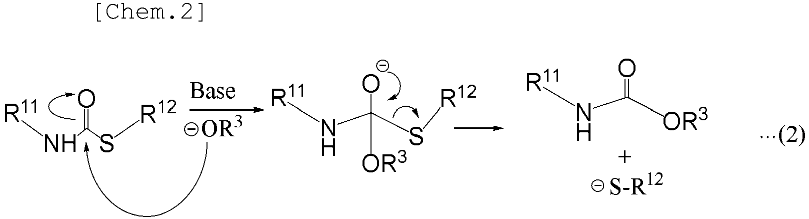

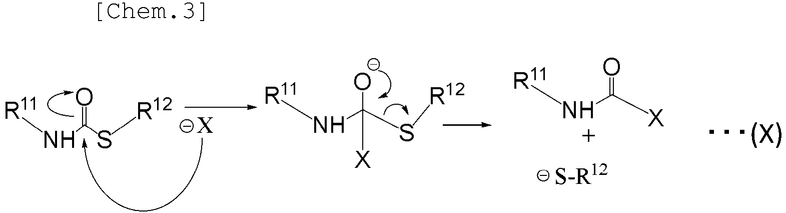

The content of the quaternary ammonium salt in the reaction system of the reaction step is not particularly limited, but is preferably 1 to 40 mass%, more preferably 2 to 30 mass%, and particularly preferably 3 to 20 mass% from the viewpoint of further improving the reactivity with the thiourethane resin.

### (Amount of Quaternary Ammonium Salt Charged)

In the reaction step, the charge mass ratio of the quaternary ammonium salt to the thiourethane resin (that is, the charge mass ratio [quaternary ammonium salt/thiourethane resin]) can be adjusted as appropriate, but is preferably 0.1 to 10, more preferably 0.2 to 9, particularly preferably 0.4 to 6.

In a case where the charge mass ratio [quaternary ammonium salt/thiourethane resin] is 0.1 or more, the generation of the polythiol composition is further promoted.

In a case where the charge mass ratio [quaternary ammonium salt/thiourethane resin] is 10 or less, the remaining of the quaternary ammonium salt in the reaction mixture can be further suppressed.

In the reaction step, the millimolar number of the quaternary ammonium salt charged relative to 1 g of the thiourethane resin is preferably 1.0 to 100.0 mmol/g, more preferably 2.0 to 50.0 mmol/g, and particularly preferably 3.0 to 25.0 mmol/g.

In the reaction step, the charge equivalent of the quaternary ammonium salt relative to the thiourethane resin (charge equivalent [quaternary ammonium salt/thiourethane resin]) is preferably 1.0 to 10.0, more preferably 1.0 to 8.0, and particularly preferably 1.0 to 6.0.

In a case where the charge equivalent [quaternary ammonium salt/thiourethane resin] is 1.0 or more, the generation of the polythiol composition is further promoted.

In a case where the charge equivalent [quaternary ammonium salt/thiourethane resin] is 10.0 or less, the remaining of the quaternary ammonium salt in the reaction mixture can be further suppressed.

Here, the charge equivalent of the quaternary ammonium salt relative to the thiourethane resin (charge equivalent [quaternary ammonium salt/thiourethane resin]) means the ratio of the number of quaternary ammonium cations in the charged quaternary ammonium salt to the total number of thiourethane bonds in the charged thiourethane resin.

### (Quaternary Ammonium Cation)

The quaternary ammonium cation is represented by NR₄⁺, and all of the four R's may be the same group, or three of the four R's may be the same group, and the other one of the four R's may be a group different from the above-described same group.

Specific examples of the quaternary ammonium cation (NR₄⁺) include tetramethylammonium ions (all of the four R's are methyl groups), tetraethylammonium ions (all of the four R's are ethyl groups), tetrabutylammonium ions (all of the four R's are butyl groups), benzyltrimethylammonium ions (three of the four R's are methyl groups and the other one is a benzyl group), and the like.

The four R's in the quaternary ammonium cation NR₄⁺ are not particularly limited, and are each independently, for example, a group selected from the group consisting of an alkyl group, an aromatic group, a heteroaryl group, and an ether-containing group.

The alkyl group is not particularly limited, but is preferably a linear, branched, or annular alkyl group having 1 to 20 carbon atoms. Here, the alkyl group may be a substituted alkyl group having a substituent or may be an unsubstituted alkyl group having no substituents. Examples of the substituent include a nitro group, a hydroxy group, and the like. The number of carbon atoms in the alkyl group represents the number of carbon atoms in a case where the alkyl group is not substituted and does not include the number of carbon atoms in a substituent in a case where the alkyl group has been substituted.

Specific examples of the unsubstituted alkyl group having no substituents include a methyl group, an ethyl group, a propyl group, a butyl group, a pentyl group, a hexyl group, a heptyl group, and the like.

The aromatic group is not particularly limited, but preferably has 4 to 20 carbon atoms. Here, the aromatic group may be a substituted aromatic group having a substituent or may be an unsubstituted aromatic group having no substituents. Examples of the substituent include a methyl group, a nitro group, a hydroxy group, and the like. The number of carbon atoms in the aromatic group represents the number of carbon atoms in a case where the aromatic group is not substituted and does not include the number of carbon atoms in a substituent in a case where the aromatic group has been substituted.

Specific examples of the unsubstituted aromatic group having no substituents include a benzyl group, a phenyl group, a naphthyl group, a phenethyl group, an anthryl group, a pyrenyl group, a thiophenyl group, and the like.

The heteroaryl group is not particularly limited, but preferably has 4 to 20 carbon atoms. Here, the heteroaryl group may be a substituted heteroaryl group having a substituent or may be an unsubstituted heteroaryl group having no substituents. Examples of the substituent include a methyl group, a nitro group, a hydroxy group, and the like. The number of carbon atoms in the heteroaryl group represents the number of carbon atoms in a case where the heteroaryl group is not substituted and does not include the number of carbon atoms in a substituent in a case where the heteroaryl group has been substituted.

Specific examples of the unsubstituted heteroaryl group having no substituents include non-condensed heteroaryl groups that are monovalent residues that are obtained by removing one hydrogen atom on a carbon atom or nitrogen atom of a monocycle or set of rings selected from pyrrole, imidazole, pyrazole, triazole, furan, thiophene, thiazole, isothiazole, oxazole, isoxazole, oxadiazole, thiadiazole, pyridine, pyrazine, pyridazine, pyrimidine, triazine, bipyrrole, terpyrrole, bithiophene, terthiophene, bipyridine, and terpyridine; condensed heteroaryl groups that are monovalent residues that are obtained by removing one hydrogen atom on a carbon atom or nitrogen atom of a compound selected from indoles, carbazoles, imidazoles, benzimidazoles, di(benzimidazoles)benzo[1,3,5]triazepines, (benzimidazo)benzimidazoles, (benzimidazo)phenanthridine, (benzoindolo)benzazepin, dibenzofuran, and dibenzothiophene; and the like.

The ether-containing group is not particularly limited, but preferably has 2 to 20 carbon atoms. Here, the ether-containing group may be a substituted ether-containing group having a substituent or may be an unsubstituted ether-containing group having no substituents. Examples of the substituent include a methyl group, a nitro group, a hydroxy group, and the like. The number of carbon atoms in the ether-containing group represents the number of carbon atoms in a case where the ether-containing group is not substituted and does not include the number of carbon atoms in a substituent in a case where the ether-containing group has been substituted.

Specific examples of the unsubstituted ether-containing groups having no substituents include a polyoxyalkylene group, a polyglycerin group, a tetrahydrofuranyl group, a benzofuranyl group, and the like.

### (Counter Anion (Counter Ion))

The counter anion X⁻ is not particularly limited, and examples thereof include halogen ions such as a fluorine ion, a chlorine ion, a bromine ion, and an iodine ion; anions represented by OR²⁻ such as hydroxide ions; and the like. These may be used singly or two or more thereof may be jointly used. R² represents a hydrogen atom or an alkyl group having 1 to 12 carbon atoms.

Among these, hydroxide ions are preferable from the viewpoint of the leaving ability.

### <<Amine Compound>>

The amine compound is not particularly limited, and examples thereof include primary amines, secondary amines, tertiary amines, and the like. These may be used singly or two or more thereof may be jointly used.

Among these, primary amines and secondary amines are preferable from the viewpoint of suppressing side reactions.

The amine compound may or may not be a cyclic amine.

As the amine compound, an amine compound containing at least one of an amino group and a monoalkylamino group and having 1 to 6 (preferably 1 to 3 and more preferably 1 or 2) amino groups and monoalkylamino groups in total is preferable.

From the viewpoint of further improving the reactivity with the thiourethane resin, the molecular weight of the amine compound is preferably 1000 or less, more preferably 500 or less, even more preferably 300 or less, and even more preferably 200 or less.

The lower limit of the molecular weight of the amine compound is, for example, 45 or more, preferably 59 or more, and more preferably 60 or more.

The molecular weight of the amine compound is not particularly limited, but is preferably 45 to 1000, more preferably 59 to 500, even more preferably 60 to 300, and even more preferably 60 to 200.

Specific examples of the amine compound include alkylamines having 2 to 10 carbon atoms; aralkylamines having 7 to 10 carbon atoms such as benzylamine; dialkylamines having 2 to 10 carbon atoms such as di-n-butylamine; alkyl diamines having 2 to 10 carbon atoms such as ethylenediamine and bis(2-aminoethyl) ether; alkyl triamines having 2 to 10 carbon atoms such as bis(2-aminoethyl) amine; hydroxyalkyl amines having 2 to 10 carbon atoms such as monoethanolamine; bis(hydroxyalkyl) amines having 2 to 10 carbon atoms such as bis(hydroxyethyl) amine; cyclic amines having 2 to 10 carbon atoms such as diazabicyclo undecene (DBU) and morpholine; alkyl (hydroxyalkyl) amines having 2 to 10 carbon atoms such as methylethanolamine and isopropylethanolamine; and the like.

The content of the amine compound in the reaction system of the reaction step is not particularly limited, but is preferably 1 to 40 mass%, more preferably 2 to 30 mass%, and particularly preferably 3 to 20 mass% from the viewpoint of further improving the reactivity with the thiourethane resin.

### (Amount of Amine Compound Charged)

In the reaction step, the charge mass ratio of the amine compound to the thiourethane resin (that is, the charge mass ratio [amine compound/thiourethane resin]) can be adjusted as appropriate, but is preferably 0.1 to 10, more preferably 0.2 to 9, and particularly preferably 0.3 to 6.

In a case where the charge mass ratio [amine compound/thiourethane resin] is 0.1 or more, the generation of the polythiol composition is further promoted.

In a case where the charge mass ratio [amine compound/thiourethane resin] is 10 or less, the remaining of the amine compound in the reaction mixture can be further suppressed.

In the reaction step, the millimolar number of the amine compound charged relative to 1 g of the thiourethane resin is preferably 1.0 to 100.0 mmol/g, more preferably 2.0 to 50.0 mmol/g, and particularly preferably 3.0 to 25.0 mmol/g.

In the reaction step, the charge equivalent of the amine compound relative to the thiourethane resin (charge equivalent [amine compound/thiourethane resin]) is preferably 1.0 to 10.0, more preferably 1.0 to 8.0, and particularly preferably 1.0 to 6.0.

In a case where the charge equivalent [amine compound/thiourethane resin] is 1.0 or more, the generation of the polythiol composition is further promoted.

In a case where the charge equivalent [amine compound/thiourethane resin] is 10.0 or less, the remaining of the amine compound in the reaction mixture can be further suppressed.

### (Primary Amines)

Specific examples of the primary amines include benzylamine, ethylenediamine, bis(2-aminoethyl)ether, bis(2-aminoethyl)amine, monoethanolamine, aniline, phenethylamine, and the like.

In a case where the reaction system further contains an alcohol as the optional component, the primary amine and the alcohol as the optional component react with each other to generate an alkoxide (R³O⁻) as shown in the following formula (3), but the primary amine itself gets involved in a nucleophilic reaction as a base as shown in the following formula (4), it is assumed that the alkoxide (R³O⁻) is not excessively generated and a side reaction can be suppressed.

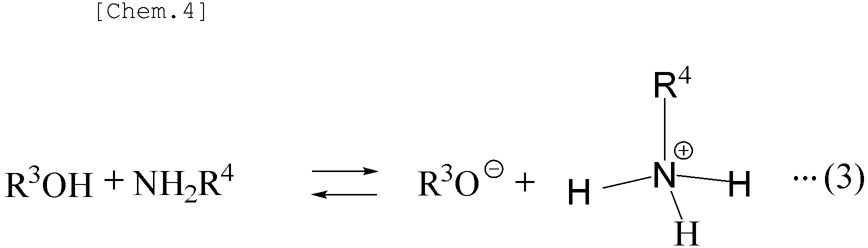

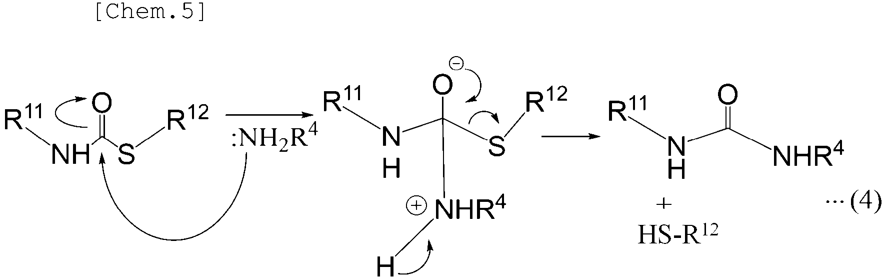

### (Secondary Amines)

Specific examples of the secondary amines include di-n-butylamine, bis(hydroxyethyl)amine, methylethanolamine, isopropyl ethanolamine, diphenylamine, and the like.

In a case where the reaction system further contains an alcohol as the optional component, the secondary amine and the alcohol as the optional component react with each other to generate an alkoxide (R³O⁻) as shown in the following formula (5), but the secondary amine itself gets involved in a nucleophilic reaction as a base as shown in the following formula (6), it is assumed that the alkoxide (R³O⁻) is not excessively generated and a side reaction can be suppressed.

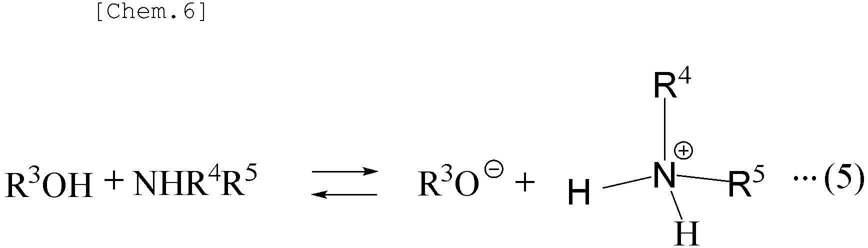

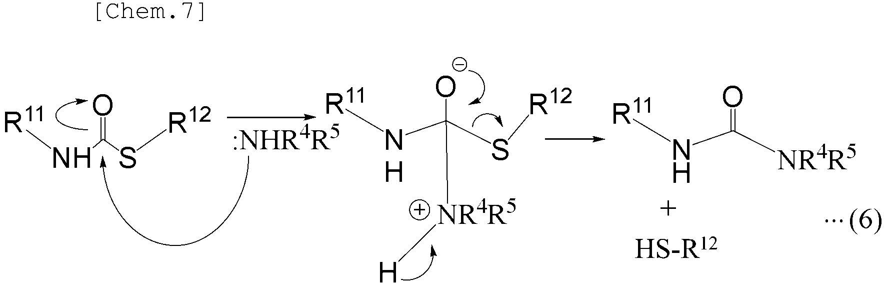

### (Tertiary Amines)

Specific examples of the tertiary amines include N,N-dimethylethanolamine, triethylamine, triisobutylamine, and the like.

In a case where the reaction system further contains an alcohol as the optional component, in order to generate "R³O⁻" on the right side of the following reaction formula (7), there is a need to increase the temperature of the reaction system in which the alcohol (R³OH) as the optional component and the tertiary amine (NR⁴R⁵R⁶) are used to shift the reaction equilibrium to the right.

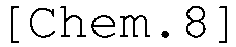

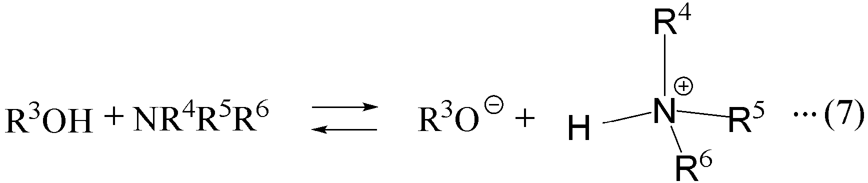

### (Cyclic Amine)

Specific examples of the cyclic amine include diazabicyclo undecene (DBU), morpholine, 1,4-diazabicyclo[2,2,2]octane, and the like.

### <Water>

The content of water in the reaction system of the reaction step is not particularly limited, but is preferably 2 to 50 mass%, more preferably 3 to 40 mass%, even more preferably 4 to 35 mass%, even more preferably 10 to 35 mass%, even more preferably 15 to 35 mass%, and particularly preferably 20 to 35 mass% from the viewpoint of further improving the reactivity with the thiourethane resin.

### (Amount of Water Charged)

In the reaction step, the mass ratio of water charged relative to the thiourethane resin (that is, the charge mass ratio [water/thiourethane resin]) can be adjusted as appropriate, but is preferably 0.1 to 10, more preferably 0.2 to 9, and particularly preferably 0.3 to 6.

In a case where the charge mass ratio [water/thiourethane resin] is 0.1 or more, the generation of the polythiol composition is further promoted.

In a case where the charge mass ratio [water/thiourethane resin] is 10 or less, the remaining of water in the reaction mixture can be further suppressed.

In the reaction step, the millimolar number of water charged relative to 1 g of the thiourethane resin is preferably 1.0 to 200.0 mmol/g, more preferably 2.0 to 150.0 mmol/g, and particularly preferably 3.0 to 130.0 mmol/g.

In the reaction step, the charge equivalent of water relative to the thiourethane resin (charge equivalent [water/thiourethane resin]) is preferably 1.0 to 10.0, more preferably 1.0 to 8.0, and particularly preferably 1.0 to 6.0.

In a case where the charge equivalent [water/thiourethane resin] is 1.0 or more, the generation of the polythiol composition is further promoted.

In a case where the charge equivalent (water/thiourethane resin) is 10.0 or less, the remaining of water in the reaction mixture can be further suppressed.

In the present invention, the reaction system contains water, which is an "environmentally friendly" solvent compared with organic solvents, which may possibly lead to development of an environmentally friendly chemical process in the future and makes a purification treatment easy.

### <Optional Component>

The optional component is not particularly limited, and examples thereof include alcohols, reaction solvents, bases other than nitrogen-containing compounds such as sodium hydroxide, and the like.

### <<Alcohols>>

In the reaction step, it is preferable to react at least one kind of alcohol as the optional component with the thiourethane resin.

It is considered that an alcohol, which is the optional component, functions as a degradation agent for the thiourethane resin.

Known alcohols can be used with no particular limitations as the alcohol, which is the optional component in the reaction system of the reaction step.

The alcohol, which is the optional component in the reaction system of the reaction step (that is, an alcohol that may be reacted with the thiourethane resin), may be only one kind or two or more kinds.

The alcohol, which is the optional component in the reaction system of the reaction step, may be a monoalcohol containing only one hydroxy group or a polyol containing two or more hydroxy groups.

The alcohol, which is the optional component in the reaction system of the reaction step, may be any of a primary alcohol such as ethanol, n-propanol, or monoethanolamine; a secondary alcohol such as isopropanol (2-propanol); or a tertiary alcohol such as t-butyl alcohol, but is preferably a lower alcohol such as methanol or ethanol from the viewpoint of a reaction in a low-temperature region.

Specific examples of the alcohol, which is the optional component in the reaction system of the reaction step, are not particularly limited and include methanol, ethanol, t-butyl alcohol, isopropanol (2-propanol), n-propanol, propylene glycol, ethylene glycol, diethylene glycol, benzyl alcohol, phenethyl alcohol, 2-octanol, 2-ethyl-1-hexanol, 1-decanol, 1-nonanol, 1-octanol, 1-heptanol, 1-hexanol, 1-pentanol, propylene glycol, ethylene glycol, monoethanolamine, and the like. These may be used singly or two or more thereof may be jointly used.

Among these, it is preferable to contain one or more alcohols miscible with water from the viewpoint of the miscibility with an aqueous solution of the nitrogen-containing compound.

Examples of the alcohols miscible with water include methanol, ethanol, t-butyl alcohol, isopropanol (2-propanol), n-propanol, propylene glycol, ethylene glycol, diethylene glycol, monoethanolamine, and the like.

In the present specification, the term "miscible" means that 10 g or more of an alcohol compound dissolves in 1 kg of water at normal temperature (25°C) and normal pressure (1 atm).

From the viewpoint of further improving the reactivity with the thiourethane resin, the molecular weight of the alcohol, which is an optional component, in the reaction step, is preferably 1000 or less, more preferably 500 or less, even more preferably 300 or less, and particularly preferably 200 or less.

The lower limit of the molecular weight of the alcohol, which is the optional component in the reaction system of the reaction step, is, for example, 30 or more.

The molecular weight of the alcohol, which is the optional component in the reaction step, is not particularly limited, but is preferably 30 to 1000, more preferably 30 to 500, even more preferably 30 to 300, and particularly preferably 30 to 200.

The alcohol, which is the optional component in the reaction step, preferably contains an alcohol having a boiling point of 60°C to 250°C (hereinafter, also referred to as "alcohol A"). In the present specification, the boiling point means a boiling point at 1 atm (101325 Pa).

The proportion of the alcohol A in the total amount of the alcohol, which is the optional component in the reaction system of the reaction step, is preferably 50 mass% to 100 mass%, more preferably 60 mass% to 100 mass%, and particularly preferably 80 mass% to 100 mass%.

The content of the alcohol, which is the optional component in the reaction system of the reaction step, is not particularly limited, but is preferably 1 to 90 mass%, more preferably 2 to 80 mass%, and particularly preferably 3 to 70 mass% from the viewpoint of further improving the reactivity with the thiourethane resin.

Hereinafter, a preferable amount of the alcohol, which is the optional component in the reaction system of the reaction step, charged will be described.

The preferable amount charged shown below also corresponds to the preferable amount of the alcohol A (that is, an alcohol having a boiling point of 60°C to 250°C) charged.

### (Amount of Alcohol, Optional Component in Reaction System of Reaction Step, Charged)

In the reaction step, the mass ratio of the alcohol, which is the optional component, charged relative to the thiourethane resin (that is, the charge mass ratio [alcohol/thiourethane resin]) can be adjusted as appropriate, but is preferably 0.10 to 20, more preferably 0.30 to 15, and particularly preferably 0.40 to 10.

In a case where the charge mass ratio [alcohol/thiourethane resin] is 0.10 or more, the generation of the polythiol composition is further promoted.

In a case where the charge mass ratio [alcohol/thiourethane resin] is 20 or less, the remaining of the alcohol in the reaction mixture can be further suppressed.

In the reaction step, the millimolar number of the alcohol, which is the optional component, charged relative to 1 g of the thiourethane resin is preferably 1.0 to 100.0 mmol/g, more preferably 3.0 to 90.0 mmol/g, and particularly preferably 5.0 to 85.0 mmol/g.

In the reaction step, the charge equivalent of the alcohol relative to the thiourethane resin (charge equivalent [alcohol/thiourethane resin]) is preferably 1.0 to 25, more preferably 1.2 to 20, and particularly preferably 1.5 to 15.

In a case where the charge equivalent [alcohol/thiourethane resin] is 1.0 or more, the generation of the polythiol composition is further promoted.

In a case where the charge equivalent [alcohol/thiourethane resin] is 25 or less, the remaining of the alcohol in the reaction mixture can be further suppressed.

Here, the charge equivalent of the alcohol relative to the thiourethane resin (charge equivalent [alcohol/thiourethane resin]) means the ratio of the number of hydroxy groups in the charged alcohol to the total number of thiourethane bonds in the charged thiourethane resin.

### (Reaction Solvent)

In the reaction step, the thiourethane resin, the nitrogen-containing compound, and the alcohol as the optional component may be reacted with one another in the presence of a reaction solvent.

The reaction solvent means a reaction solvent other than the alcohol as the optional component and water, and examples thereof include hydrocarbons having 5 to 12 (preferably 6 to 10, more preferably 7 to 9) carbon atoms, ethers having 4 to 12 carbon atoms, ketones having 3 to 12 carbon atoms, esters having 4 to 12 carbon atoms, nitrile having 2 to 12 carbon atoms, and the like. These may be used singly or two or more thereof may be jointly used.

The hydrocarbon is preferably hexane, heptane, octane, nonane, decane, xylene, mesitylene, or toluene, more preferably heptane, octane, nonane, xylene, mesitylene, or toluene, and particularly preferably xylene or toluene.

The ether is preferably diethyl ether, tetrahydrofuran, 2-methyltetrahydrofuran, dimethoxyethane, or 1,4-dioxane and more preferably dimethoxyethane.

The ketone is preferably acetone, methyl ethyl ketone, methyl isobutyl ketone, or 2-octanone and more preferably methyl isobutyl ketone.

The ester is preferably ethyl acetate, butyl acetate, or pentyl acetate and more preferably pentyl acetate.

The nitrile is preferably acetonitrile or propionitrile and more preferably acetonitrile.

The content of the reaction solvent in the reaction system of the reaction step is not particularly limited, but is preferably 95 mass% or less, more preferably 90 mass% or less, and particularly preferably 85 mass% or less from the viewpoint of further improving the reactivity with the thiourethane resin.

### <Reaction Temperature>

The reaction temperature of the thiourethane resin, the nitrogen-containing compound, and the alcohol as the optional component in the reaction step can be adjusted as appropriate.

In the reaction step, the thiourethane resin, the nitrogen-containing compound, water, and the alcohol as the optional component are preferably reacted with one another under a temperature condition (that is, a reaction temperature) of 15°C to 110°C (more preferably 30°C to 105°C, and particularly preferably 40°C to 100°C).

In a case where the reaction temperature is 15°C to 110°C, it is possible to further improve the purity of a polythiol component as a main component in the polythiol composition as a target substance (that is, the content of the main component relative to the total amount of the polythiol composition).

In addition, in the reaction step, the reaction may be performed under a pressurized condition. In a case where the reaction has been performed under a pressurized condition, the reaction time may be shortened.

### <Reaction Time>

The reaction time among the thiourethane resin, the nitrogen-containing compound, water, and the alcohol as the optional component in the reaction step can be adjusted as appropriate, but is preferably 0.10 to 5.50 hours, more preferably 0.50 to 5.00 hours, even more preferably 1.00 to 4.50 hours, and particularly preferably 1.00 to 4.00 hours.

### <Polythiol Composition>

In the present disclosure, the polythiol composition means a composition containing at least one polythiol compound, and may contain a different component such as a polyisocyanate compound or a polyamine compound.

In the present disclosure, the polythiol compound that is contained in the polythiol composition is also referred to as "polythiol component."

The polythiol composition preferably contains at least one kind of polythiol compound as a main component. Here, "the polythiol composition contains at least one kind of polythiol compound as a main component" means that the total content of the at least one polythiol compound relative to the total amount of the polythiol composition is 50 mass% or more. The total content of the at least one polythiol compound relative to the total amount of the polythiol composition is preferably 60 mass% or more, more preferably 70 mass% or more, and particularly preferably 80 mass% or more.

Examples of the polythiol composition as the target substance include polythiol compositions containing a known polythiol compound.

The polythiol composition as the target substance and the polythiol composition as a raw material of the thiourethane resin as the starting material do not need to be completely the same as each other.

However, from the viewpoint of the performance of the thiourethane resin that is produced with the polythiol composition as the target substance, it is preferable that the kind of the polythiol component as the main component in the polythiol composition as the target substance is the same as the kind of the polythiol component as the main component in the polythiol composition as a raw material. In this case, it is possible to produce an optical material B (optical material containing a thiourethane resin) having performance comparable to that of the optical material A using, for example, chips (thiourethane resin) generated during the production of the optical material A as a raw material.

The polythiol composition as the target substance may contain the same polythiol component as the main component as the polythiol composition as a raw material of the thiourethane resin as a starting material and may have a reduced content of an impurity.

In the polythiol composition as the target substance, in a case where the content of the impurity has been reduced, an increase in the viscosity of the polythiol composition is suppressed, and the polythiol composition can have an advantage of a long pot life.

The application of the polythiol composition as the target substance is not particularly limited.

The polythiol composition as the target substance can be used for, for example, the production of thiourethane resins.

Specific applications of the polythiol composition as the target substance include polythiol compositions for producing optical materials (for example, spectacle lenses).

In other words, specific examples of the method for producing a polythiol composition of the present disclosure include methods for producing a polythiol composition for the production of optical materials. In these specific examples, in the case of using chips containing a thiourethane resin generated during the production of an optical material as a starting material, an effective use (that is, recycling) of the materials (the thiourethane resin and the polythiol composition as raw materials thereof) is effectively achieved.

In addition, in the reaction step in the present disclosure, a polythiol composition is obtained by reacting the thiourethane resin and the nitrogen-containing compound in the presence of water, whereby a polythiol composition in which the polythiol component as the main component has a high purity can be obtained compared with known methods (for example, a method in which a polythiol composition is obtained by a reaction between a thiourethane resin and sodium hydroxide).

Accordingly, even in a case where the polythiol composition as the target substance is used for the production of an optical material (for example, a lens), it is possible to obtain an optical material having favorable performance.

Examples of the performance of the optical material include optical properties (for example, the refractive index and/or the Abbe number), heat resistance, specific gravity, and the like.

### (Polythiol Compound)

The polythiol compound is not particularly limited as long as the polythiol compound has two or more thiol groups (also known as mercapto groups).

Regarding the polythiol compound, the above-described known publications (that is, Japanese Patent Application Publication No. S63-46213, Japanese Patent Application Publication No. H02-270859, Japanese Patent Application Publication No. H07-252207, WO 2008/047626, and the like) can be referred to as appropriate.

The polythiol compound is not particularly limited, and examples thereof suitably include 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, pentaerythritol tetrakis(2-mercaptoacetate), pentaerythritol tetrakis(3-mercaptopropionate), 2,5-dimercaptomethyl-1,4-dithiane, bis(2-mercaptoethyl)sulfide, diethyleneglycol bis(3-mercaptopropionate), and the like (hereinafter, also referred to as "polythiol component A"). These may be used singly or two or more thereof may be jointly used.

The polythiol composition more preferably contains the polythiol component A as a main component. In this case, the polythiol composition may contain at least one different component other than the polythiol component A (for example, a different polythiol compound, components other than the polythiol compound, or the like).

The different polythiol compound is not particularly limited, and examples thereof include methanedithiol, 1,2-ethanedithiol, 1,2,3-propanetrithiol, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl)sulfide, 2,5-dimercapto-1,4-dithioane, 2,5-dimercaptomethyl-2,5-dimethyl-1,4-dithioane, 1,1,3,3-tetrakis(mercaptomethylthio)propane, 1,1,2,2-tetrakis(mercaptomethylthio)ethane, 4,6-bis(mercaptomethylthio)-1,3-dithiane, and the like. These may be used singly or two or more thereof may be jointly used.

### (Polyisocyanate Compound)

The polyisocyanate compound may be a compound having two or more isocyanate groups.

The polyisocyanate compound is not particularly limited, and examples thereof include pentamethylene diisocyanate, hexamethylene diisocyanate, m-xylylene diisocyanate, p-xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylethane diisocyanate, phenylene diisocyanate, and the like. These may be used singly or two or more thereof may be jointly used.

Among these, m-xylylene diisocyanate, 2,5-bis(isocyanatomethyl)bicyclo-[2.2.1]-heptane, 2,6-bis(isocyanatomethyl)bicyclo-[2.2.1] heptane are preferable.

### (Polyamine Compound)

The polyamine compound needs to be a compound having two or more amino groups.

The polyamine compound is not particularly limited, and examples thereof include pentamethylenediamine, hexamethylenediamine, m-xylylenediamine, p-xylylenediamine, isophorone diamine, bis(aminomethyl)cyclohexane, bis(aminocyclohexyl)methane, 2,5-bis(aminomethyl)bicyclo-[2.2.1]-heptane, 2,6-bis(aminomethyl)bicyclo-[2.2.1]-heptane, tolylenediamine, 4,4'-diphenylmethandiamine, phenylenediamine, and the like. These may be used singly or two or more thereof may be jointly used.

Among these, m-xylylenediamine, 2,5-bis(aminomethyl)bicyclo-[2.2.1] heptane, and 2,6-bis(aminomethyl)bicyclo-[2.2.1] heptane are preferable.

### (Preferable Aspects of Step of Generating Polythiol Composition)

The step of generating a polythiol composition is preferably a step of reacting the thiourethane resin, the nitrogen-containing compound, and the alcohol as the optional component in the presence of water to generate the polythiol compound and an optional polyamine compound as the polythiol composition.

In such a preferable aspect, a degradation reaction occurs in which the thiourethane resin is degraded into the polythiol compound and the optical polyamine compound by the alcohol as a degradation agent. The above-described degradation reaction is an alcoholysis reaction.

### (Resin Mixture Containing Thiourethane Resin)

The step of generating a polythiol composition may be a step of bringing a resin mixture containing the thiourethane resin, the nitrogen-containing compound, and the alcohol as the optional component into contact with one another in the presence of water, thereby reacting the thiourethane resin in the resin mixture, the nitrogen-containing compound, and the alcohol as the optional component with one another in the presence of water to generate a polythiol composition.

The resin mixture containing the thiourethane resin further contains a component other than the thiourethane resin.

Examples of the component other than the thiourethane resin include resins other than the thiourethane resin, inorganic materials for making a lens (for example, glass), and the like.

The resins other than the thiourethane resin are not particularly limited, and examples thereof include a hybrid material of a thiourethane resin and a urethane resin produced by adding a polyol to the raw material at the time of producing the thiourethane resin; a hybrid material of a thiourethane resin and a urea resin produced by adding a polyamine compound to the raw material at the time of producing the thiourethane resin; a polyolefin film that protects the surface of a resin molded body for making a spectacle lens; a hard coat or primer coat that protects the surface of a resin molded body for making a spectacle lens; a polishing agent that is used at the time of polishing a resin molded body for making a spectacle lens; a resin material for fixing a resin molded body for making a spectacle lens at the time of cutting the resin molded body; tape or tape glue that is used to fix a glass mold that is used at the time of fabricating a resin molded body for making a spectacle lens; and the like.

Specific examples of the resins other than the thiourethane resin are not particularly limited and suitably include polycarbonate resins, polyallyl carbonate resins, acrylic resins, urethane resins, episulfide resins, and the like.

The resin mixture containing the thiourethane resin is preferably a resin mixture collected in at least one of a manufacturing process of a spectacle lens, a manufacturing process of spectacles, and a disposal process of spectacles.

The manufacturing process of a spectacle lens, the manufacturing process of spectacles, and the disposal process of spectacles are as described above.

The resin mixture containing the thiourethane resin preferably contains chips containing the thiourethane resin.

### (Reaction Mixture Containing Polythiol Composition)

The step of generating a polythiol composition may be a step of reacting the thiourethane resin, the nitrogen-containing compound, and the alcohol as the optional component in the presence of water to generate a polythiol composition, and obtaining a reaction mixture containing the polythiol composition as the target substance.

The reaction mixture may contain a polythiol composition as a main product that is generated by alcoholysis and a different component other than the polythiol composition.

Examples of the different component other than the polythiol composition in the reaction mixture include byproducts that are generated by alcoholysis (for example, polycarbamates), the above-described reaction solvents, residues of the raw materials (the thiourethane resin, the alcohol as the optional component, and/or the nitrogen-containing compound), an impurity contained in the raw materials, and the like.

### [[Separation Step]]

The method for producing the polythiol composition may include a separation step of separating the polythiol composition as the target substance from the reaction mixture containing the above-described polythiol composition.

A separation method in the separation step is not particularly limited, and examples thereof include known methods such as filtration, decantation, extraction, distillation, drying (including vacuum drying), and purification (for example, column chromatogram). These may be used singly or two or more thereof may be jointly used.

The separation step preferably includes filtering the reaction mixture containing the polythiol composition obtained in the reaction step to obtain a filtrate containing the polythiol composition.

According to this aspect, it is easier to remove a solid content that is contained in the reaction mixture.

Examples of a method for separating the polythiol compound in the polythiol composition include methods in which the polythiol compound is extracted with an organic or inorganic solvent capable of dissolving the polythiol compound.

As a method for purifying the polythiol compound, common purification methods such as column purification, distillation purification, recrystallization purification, and salt extraction are used.

Examples of a method for separating the polyamine compound in the polythiol composition include methods in which the polyamine compound is extracted with an organic or inorganic solvent capable of dissolving the polyamine compound.

As a method for purifying the polyamine compound, common purification methods such as column purification, distillation purification, recrystallization purification, and salt extraction are used.

In a case where the step of generating a polythiol composition is the above-described step of generating a polythiol compound and a polyamine compound, the separation step preferably includes at least one of obtaining a filtrate containing the polythiol compound as a filtrate and obtaining a mixture containing a polyamine compound derivative as a filtered substance by filtering the reaction mixture containing the polythiol compound and the polyamine compound derivative.

In a case where the separation step includes obtaining a filtrate containing the polythiol compound as a filtrate, the polythiol compound as the polythiol composition can be obtained by separating the polythiol compound from the filtrate.

As one example of the separation step in this case, there is a method including filtering the reaction mixture containing the polythiol compound and the polyamine compound as the polythiol composition to obtain a filtrate including the polythiol compound, adding a base containing an alkali metal and then adding water to the filtrate containing the polythiol compound to perform extraction, thereby obtaining an aqueous extract containing an alkali metal salt of the polythiol compound, adding an acid to the aqueous extract containing an alkali metal salt of the polythiol compound to obtain an aqueous liquid containing the polythiol compound, adding a hydrocarbon having 5 to 12 carbon atoms as an extraction solvent to the aqueous liquid containing the polythiol compound to perform extraction, thereby obtaining an extract containing the polythiol compound, and separating the polythiol compound from the extract containing the polythiol compound.

In this example, first, the polythiol compound in the filtrate containing the polythiol compound is converted into an alkali metal salt and extraction is then performed with water, thereby obtaining an aqueous extract containing the alkali metal salt of the polythiol compound. Next, an acid is added thereto, thereby returning the alkali metal salt of the polythiol compound to the polythiol compound. The polythiol compound is extracted from the obtained aqueous liquid containing the polythiol compound with the extraction solvent, and an extract containing the polythiol compound is obtained. The polythiol compound is separated from the obtained extract containing the polythiol compound.

According to this example, even in a case where a large amount of a different component other than the polythiol compound is contained in the filtrate containing the polythiol compound, a polythiol compound in which the polythiol component as the main component has a higher purity can be obtained.

The alkali metal in the base containing an alkali metal is not particularly limited, but is preferably sodium, potassium, or lithium and more preferably sodium or potassium.

The base containing an alkali metal is not particularly limited, and examples thereof include sodium methoxide, sodium ethoxide, sodium propoxide, sodium hydroxide, potassium hydroxide, lithium hydroxide, and the like.

The base containing an alkali metal can be added to the filtrate, if necessary, in the form of an alcohol solution (a methanol solution, an ethanol solution, or the like).

The acid that is added to the aqueous extract containing an alkali metal salt of the polythiol compound is not particularly limited, and examples thereof include hydrochloric acid, carbonic acid, nitric acid, sulfuric acid, acetic acid, formic acid, oxalic acid, and the like.

The extraction solvent may be only one kind or two or more kinds.

A preferable aspect of the extraction solvent is the same as the above-described preferable aspect of the reaction solvent.

Here, the reaction solvent and the extraction solvent may be the same as or different from each other.

### [[Other Steps]]

The method for producing a polythiol composition may include other steps other than the above-described steps, if necessary.

Examples of the other steps include a classification step; a sieving step; a washing step; a crushing (pulverizing) step; and the like.

### <Classification Step>

The method for producing a polythiol composition may further include, prior to the reaction step of generating a polythiol composition, a classification step of classifying the chips containing the thiourethane resin, thereby obtaining a powder having a smaller average particle size (for example, the number-average value of equivalent circle diameters) than the chips and containing a thiourethane resin (that is, chips having a reduced average particle size).

In the reaction step of generating a polythiol composition in the case of including this classification step, the powder, the nitrogen-containing compound, and the alcohol as the optional component are brought into contact with one another in the presence of water, thereby reacting the thiourethane resin in the powder, the nitrogen-containing compound, and the alcohol as the optional component with one another in the presence of water.

In a case where the method for producing a polythiol composition includes the classification step, in the reaction step, a powder composed of particles having a small particle size (that is, average particle size), the nitrogen-containing compound, and the alcohol as the optional component are brought into contact with one another in the presence of water, and it is thus possible to further improve the reaction efficiency of the thiourethane resin in the powder, the nitrogen-containing compound, and the alcohol as the optional component in the presence of water.

Examples of the average particle size include a number average particle size.

Examples of the particle size include an equivalent circle diameter.

Examples of a classification method include sieving, centrifugation, and the like.

Regarding an aspect in which sieving is performed as classification, the following sieving step can be referred to.

### <Sieving Step>

The method for producing a polythiol composition may include, prior to the reaction step of generating a polythiol composition, a sieving step of sieving the chips containing the thiourethane resin, thereby obtaining a powder including the thiourethane resin that has passed through a sieve (that is, chips that has passed through the sieve).

In the reaction step of generating a polythiol composition in the case of including this sieving step, the powder, the nitrogen-containing compound, and the alcohol as the optional component are brought into contact with one another in the presence of water, thereby reacting the thiourethane resin in the powder, the nitrogen-containing compound, and the alcohol as the optional component with one another in the presence of water.

In a case where the method for producing a polythiol composition includes the sieving step, in the reaction step, a powder composed of particles having a small particle size, the nitrogen-containing compound, and the alcohol as the optional component are brought into contact with one another in the presence of water, and it is thus possible to further improve the reaction efficiency of the thiourethane resin, the nitrogen-containing compound, and the alcohol as the optional component.

### The sieve is not particularly limited.

The nominal aperture size of the sieve regulated by JIS Z-8801-1: 2019 is, for example, 0.1 to 20 mm, preferably 0.1 to 10 mm, more preferably 0.1 to 5 mm, even more preferably 0.1 to 2 mm, even more preferably 0.3 to 2 mm, and particularly preferably 0.5 to 1.5 mm.

### <Washing Step>

The method for producing a polythiol composition may include, prior to the reaction step of generating a polythiol composition, a washing step of washing the thiourethane resin powder (that is, the powder containing the thiourethane resin) with a hydrocarbon having 5 to 12 carbon atoms as a washing solvent.

In the reaction step of generating a polythiol composition in the case of including this washing step, the powder washed in the washing step, the nitrogen-containing compound, and the alcohol as the optional component are brought into contact with one another in the presence of water, thereby reacting the thiourethane resin in the powder, the nitrogen-containing compound, and the alcohol as the optional component with one another in the presence of water. This makes it possible to obtain a polythiol composition in which the polythiol component as the main component has a higher purity.

Particularly, in the method for producing a polythiol composition, in a case where the chips containing the thiourethane resin is used as a starting material, it is possible to effectively remove oil that has been derived from a cutting machine and attached to the chips by the washing step, and a polythiol composition in which the polythiol component as the main component has a higher purity can be thus obtained.

The hydrocarbon as the washing solvent may be used singly or two or more thereof may be jointly used.

A preferable aspect of the hydrocarbon as the washing solvent is the same as the above-described preferable aspect of the hydrocarbon as the reaction solvent.

Here, the reaction solvent and the washing solvent may be the same as or different from each other.

A washing method in the washing step is not particularly limited, and it is possible to apply known methods such as a method in which the washing solvent is added to and mixed with the thiourethane resin powder.

In a case where the method for producing a polythiol composition includes the above-described sieving step and washing step, the sieving step and the washing step are preferably performed in this order. In this case, since there is no need to wash chips that have not passed through the sieve, the amount of the washing solvent used can be further reduced.

### <Crushing (Pulverizing) Step>

The method for producing a polythiol composition may include, prior to the reaction step of generating a polythiol composition, a crushing (pulverizing) step of crushing and/or pulverizing the thiourethane resin.

A crushing (pulverizing) method in the crushing (pulverizing) step is not particularly limited, and known methods can be applied.

### [Method for Producing Polymerizable Composition]

A method for producing a polymerizable composition of the present disclosure includes a step of producing a polythiol composition by the method for producing a polythiol composition of the present disclosure, and a step of mixing a polythiol composition containing at least the produced polythiol composition and a polyisocyanate to obtain a polymerizable composition containing the polythiol composition, the polyisocyanate, and a different optional component, and may further includes a different step if necessary.

In the method for producing a polymerizable composition of the present disclosure, a polythiol composition is produced using a thiourethane resin (for example, a thiourethane resin in a ground powder of a molded body of the thiourethane resin) as a starting material in the step of producing a polythiol composition, and a polymerizable composition containing the polythiol composition produced above and a polyisocyanate compound is produced in the step of obtaining a polymerizable composition.

The obtained polymerizable composition can be used again for the production of a thiourethane resin.

Therefore, in the method for producing a polymerizable composition, an effective use (that is, recycling) of the materials (that is, the thiourethane resin and the polythiol composition, which is a raw material thereof) is achieved.

In addition, as described above, according to the method for producing a polythiol composition, compared with known methods (for example, a method in which a polythiol composition is obtained by a reaction between a thiourethane resin and sodium hydroxide), a polythiol composition in which a polythiol component as a main component has a high purity can be obtained. According to the polymerizable composition obtained by the method for producing a polymerizable composition, it is possible to produce resins being excellent in terms of various performances [for example, optical properties (for example, refractive index and/or Abbe's number), heat resistance, specific gravity, and the like].

Therefore, the polymerizable composition that is obtained by the method for producing a polymerizable composition of the present disclosure is particularly suitable as a composition for the production of a thiourethane resin for optical materials.

### [[Step of Producing Polythiol Composition]]

Regarding the step of producing a polythiol composition, the above-described method for producing a polythiol composition of the present disclosure can be referred to as appropriate.

### [[Step of Obtaining Polymerizable composition]]

In the step of obtaining a polymerizable composition, at least the polythiol composition and a polyisocyanate compound are mixed together, thereby obtaining a polymerizable composition containing the polythiol composition and the polyisocyanate compound.

A preferable aspect of the polyisocyanate compound that is used in the step of obtaining a polymerizable composition is the same as the preferable aspect of "polyisocyanate compound" described in the section of "polythiol composition."

In the step of obtaining a polymerizable composition, the mixing ratio between the polythiol composition and the polyisocyanate compound is not particularly limited.

In the step of obtaining a polymerizable composition, the ratio of the mass of the polythiol composition charged to the mass of the polyisocyanate compound charged (that is, the charge mass [polythiol composition/polyisocyanate compound]) is preferably 0.10 to 10.0, more preferably 0.20 to 5.00, even more preferably 0.50 to 1.50, and particularly preferably 0.70 to 1.30.

In addition, the molar ratio (mercapto group/isocyanate group) of a mercapto group of the polythiol compound to an isocyanate group of the polyisocyanate compound that are contained in the polythiol composition is preferably 0.5 to 3.0, more preferably 0.6 to 2.0, and particularly preferably 0.8 to 1.3.

In the step of obtaining a polymerizable composition, the total charge mass of the polythiol composition and the polyisocyanate compound is not particularly limited, but is preferably 60 mass% or more, more preferably 80 mass% or more, and particularly preferably 90 mass% or more of the total amount of the polymerizable composition to be produced.

In the step of obtaining a polymerizable composition, at least the polythiol composition and a polyisocyanate compound are mixed together, but the polythiol composition and polyisocyanate compound and a different component may also be mixed together, if necessary.

In addition, in the step of obtaining a polymerizable composition, after at least the polythiol composition and the polyisocyanate compound are mixed together, the different component may be added to the mixture.

The different component is not particularly limited, and examples thereof include a polymerization catalyst, an internal mold release agent, a resin modifier, a chain extender, a cross-linking agent, a radical scavenger, a light stabilizer, an ultraviolet absorber, an antioxidant, an oilsoluble dye, a filler, an adhesion improving agent, an antibacterial agent, an antistatic agent, a dye, a fluorescent brightener, a fluorescent pigment, an inorganic pigment, and the like.

### <Polymerization Catalyst>

The polymerization catalyst is not particularly limited, and examples thereof include tertiary amines, inorganic or organic acid salts of tertiary amines, metal compounds such as dimethyltin dichloride, quaternary ammonium salts, organic sulfonic acids, and the like. These may be used singly or two or more thereof may be jointly used.

### <Internal Mold Releasing Agent>

The internal mold release agent is not particularly limited, and examples thereof include acidic phosphate esters such as phosphate monoesters and phosphate diesters; and the like. These may be used singly or two or more thereof may be jointly used.

### <Resin Modifier>

The resin modifier is not particularly limited, and examples thereof include episulfides; epoxy; organic acids; anhydrides of organic acids; (meth)acrylates; olefins; and the like. These may be used singly or two or more thereof may be jointly used.

### (Meth)acrylate means at least one of acrylate and methacrylate.

In the step of obtaining a polymerizable composition, the above-described components can be mixed together according to a conventional method, and a mixing method is not particularly limited.

### [Method for Producing Resin]

A method for producing a resin of the present disclosure includes a step of producing a polymerizable composition by the above-described method for producing a polymerizable composition and a step of curing the polymerizable composition to obtain a resin.

The method for producing a resin of the present disclosure may include a different step, if necessary.

According to the method for producing a resin of the present disclosure, the same effect as that of the above-described method for producing a polymerizable composition of the present disclosure is achieved.

A resin that is produced by the method for producing a resin of the present disclosure is a thiourethane resin, but will be simply referred to as "resin" in the present disclosure in order to distinguish the resin from the thiourethane resin that is one of the starting materials of the polythiol composition.

In the step of obtaining a resin, the above-described polymerizable composition is cured, thereby obtaining a resin.

The polymerizable composition can be cured by polymerizing monomers in the polymerizable composition (specifically, the polythiol composition and the polyisocyanate compound, which will also be true below). As a pretreatment for the polymerization, a treatment such as filtration or degasification may be performed on the polymerizable composition.

The polymerization conditions (for example, the polymerization temperature, the polymerization time, and the like) for polymerizing the monomers in the polymerizable composition are appropriately set in consideration of the composition of the composition, the kinds and amounts of the monomers used in the composition, the kind and amount of the polymerization catalyst used in the composition, the properties of a mold described below in the case of using a mold to be described below, and the like.

The polymerization temperature is not particularly limited, but is preferably -50°C to 150°C and more preferably 10°C to 150°C.

The polymerization time is not particularly limited, but is preferably 1 to 200 hours and more preferably 1 to 80 hours.

In the step of obtaining a resin, a treatment such as annealing may be performed on a polymer obtained by the polymerization of the monomers to obtain a resin.

The annealing temperature is not particularly limited, but is preferably 50°C to 150°C, more preferably 90°C to 140°C, and particularly preferably 100°C to 130°C.

### [[Method for Producing Molded Body]]

A method for producing a molded body is a method for producing a molded body containing a resin, includes a step of producing a polymerizable composition by the above-described method for producing a polymerizable composition and a step of curing the polymerizable composition to obtain a molded body containing a resin, and may include a different step if necessary.

According to the method for producing a molded body, the same effect as that of the above-described method for producing a polymerizable composition is achieved.

In the step of obtaining a molded body containing a resin, the polymerizable composition is cured, thereby obtaining a molded body containing a resin.

Regarding preferable conditions for the curing of the polymerizable composition, that is, the polymerization of the monomers in the polymerizable composition, the section of "method for producing a resin" can be referred to as appropriate.

One example of the polymerization in the present step includes cast polymerization.

In cast polymerization, first, the above-described polymerizable composition is injected between molding molds held by a gasket, tape, or the like. At this time, a defoaming treatment, a filtration treatment, or the like may be performed, if necessary.

Next, the monomers in the polymerizable composition injected between the molding molds are polymerized, thereby curing the composition between the molding molds to obtain a cured product. Next, the cured product is removed from the molding molds to obtain a molded body containing a resin.

The monomers may be polymerized by heating the polymerizable composition. This heating can be performed, for example, using a heating device equipped with a mechanism for heating an object to be heated in an oven, water, or the like.

### [[Method for Producing Optical Material and Method for Producing lens]]

A method for producing an optical material (for example, a lens) is a method for producing an optical material including a molded body containing a resin (for example, a lens), includes a step of producing a polymerizable composition by the above-described method for producing a polymerizable composition and a step of curing the polymerizable composition to obtain a molded body containing a resin, and may include a different step, if necessary.

According to the method for producing an optical material, the same effect as that of the above-described method for producing a polymerizable composition is achieved.

The method for producing an optical material is an application of the method for producing a molded body.

For example, in the method for producing a molded body, the shape of the molding mold that is used for the above-described cast polymerization is suitably selected, whereby it is possible to obtain a molded body applicable to an optical material (for example, a lens).

Examples of the optical material include lenses (for example, spectacle lenses, camera lenses, and polarized lenses), light-emitting diodes (LEDs), and the like.

The method for producing an optical material (for example, a lens) may include a step of forming a coating layer on one surface or both surfaces of the molded body containing a resin.

Specific examples of the coating layer include a primer layer, a hard coat layer, an anti-reflection layer, an antifog coating layer, an anti-fouling layer, a water-repellent layer, and the like.

These coating layers may be each formed singly or a plurality of the coating layers may be formed in multiple layers. In the case of forming the coating layers on both surfaces, the same coating layer may be formed on each surface, or different coating layers may be formed.

A component of the coating layer can be selected as appropriate depending on the purpose.

Examples of the component of the coating layer include resins such as a urethane resin, an epoxy resin, a polyester resin, a melamine resin, and a polyvinyl acetal resin; an infrared absorber; a light stabilizer; an antioxidant; a photochromic compound; a dye; a pigment; an antistatic agent; and the like.

Regarding the spectacle lens and the coating layer, for example, the description of known publications, such as WO 2017/047745 can be referred to as appropriate.

### [[Polymerizable Composition]]

The polymerizable composition contains a polythiol composition obtained by the method for producing a polythiol composition and a polyisocyanate compound.

The polymerizable compositions can be produced by the above-described method for producing a polymerizable composition.

According to the polymerizable composition, the same effect as that of the above-described method for producing a polymerizable composition is achieved.

Regarding a preferable aspect of the polymerizable composition, the above-described method for producing a polymerizable composition can be referred to as appropriate.

Here, the charge mass [polythiol composition/polyisocyanate compound] can be replaced by the content mass ratio [polythiol composition/polyisocyanate compound], and the total charge mass of the polythiol composition and the polyisocyanate compound can be replaced by the total content mass of the polythiol composition and the polyisocyanate compound.

### [[Resin, Molded Body, Optical Material (For Example, Lens)]]

A resin is a cured product of the above-described polymerizable composition.

A molded body is a molded body containing the above-described resin.

An optical material (for example, a lens) is an optical material (for example, a lens) containing the above-described resin.

According to the resin, the molded body, and the optical material (for example, a lens), the same effect as that of the above-described method for producing a polymerizable composition is achieved.

The resin, the molded body, and the optical material (for example, the lens) can be produced by the method for producing a resin, the method for producing a molded body, and the method for producing an optical material (for example, a lens), which have been described above, respectively.

Regarding preferable aspects of the resin, the molded body, and the optical material (for example, the lens), the preferable aspects of the method for producing a resin, the method for producing a molded body, and the method for producing an optical material (for example, a lens) can be referred to.

### <Preferable Performance of Resin or Molded Body>

The glass transition temperature Tg of the resin (or the molded body) is not particularly limited, but is preferably 70°C or higher, more preferably 80°C or higher, and particularly preferably 85°C or higher from the viewpoint of heat resistance.

The upper limit of the glass transition temperature Tg is not particularly limited, and may be 130°C or lower; 120°C or lower, or may be 110°C or lower.

The glass transition temperature Tg is not particularly limited, but is preferably 70°C to 130°C, more preferably may be 80°C to 120°C, and particularly preferably 85°C to 110°C.

The refractive index (ne) of the resin (or the molded body) is not particularly limited, but is preferably 1.500 or more, more preferably 1.540 or more, and particularly preferably 1.590 or more from the viewpoint of application to optical materials.

The upper limit of the refractive index (ne) is not particularly limited, but is preferably 1.750.

The refractive index (ne) is not particularly limited, but is preferably 1.500 to 1.750, more preferably 1.540 to 1.750, and particularly preferably 1.590 to 1.750.

The Abbe's number of the resin (or the molded body) is not particularly limited, but is preferably 28 or more and more preferably 30 or more from the viewpoint of application to optical materials.

The upper limit of the Abbe's number is not particularly limited, but is preferably 50 and more preferably 45.

The Abbe's number is not particularly limited, but is preferably 28 to 50 and more preferably 30 to 45.

The specific gravity of the resin (or the molded body) is not particularly limited, but is preferably 1.10 or more and more preferably 1.20 or more from the viewpoint of application to optical materials.

The upper limit of the specific gravity is not particularly limited, but is preferably 1.50 and more preferably 1.40.

The specific gravity is not particularly limited, but is preferably 1.10 to 1.50 and more preferably 1.20 to 1.40.

In the present disclosure, regarding examples, contents, and various physical properties of each of the above components, matters described in the detailed description of the invention as illustrations or preferable ranges may be optionally combined together.

In addition, as long as the compositions described in the examples can be adjusted to the compositions described in the detailed description of the invention, it is possible to perform the invention in the same manner as the examples throughout the entire claimed composition scopes.

### Examples

Hereinafter, the present disclosure will be further described using Examples. However, the present disclosure is not limited to an embodiment shown in Examples.

### [Production Example 1]

### <Production of Molded Body Containing Thiourethane Resin>

Dimethyltin dichloride, which is a polymerization catalyst, (0.0075 parts by mass relative to 100 parts by mass of the total amount of the following polyisocyanate compound and the following polythiol composition), JP-506H, which is a mold release agent, (manufactured by Johoku Chemical Co., Ltd.; acidic phosphate ester) (0.15 parts by mass relative to 100 parts by mass of the total amount of the following polyisocyanate compound and the following polythiol composition), m-xylylene diisocyanate (XDI), which is a polyisocyanate compound, (49.6 parts by mass) were added into a flask equipped with a stirrer. After the various additives were sufficiently dissolved by stirring, as a polythiol component, a polythiol composition containing, as main components, 4,8-dimercaptomethyl-1,11-dimercapto-3,6,9-triathiane, 4,7-dimercaptomethyl-1,11-dimercapto-3,6,9-trithiaane, and 5,7-dimercaptomethyl-1,11-dimercapto-3,6,9-trithiane (50.4 parts by mass) was added thereto and mixed therewith, and a polymerizable composition, which was a transparent homogeneous solution, was obtained. This polymerizable composition was degassed at 300 Pa for 30 minutes or longer, and then filtered through a polytetrafluoroethylene (PTFE) filter having a pore size of 5 µm. Subsequently, the mixture was injected into a mold that had a desired lens shape and was made up of a glass mold and a gasket. The mold into which the polymerizable composition had been injected was polymerized in an oven in a temperature range from 10°C to 120°C for 24 hours depending on the shape of the lens. The molding die was taken out of the oven and removed to obtain a lens molded body for spectacles made of a resin for an optical component. The obtained molded body was annealed at 120°C for two hours.

### <Production of Thiourethane Resin Powder>

The molded body obtained above was cut, thereby producing a lens. Chips generated at this time were collected and sieved with a sieve having a nominal aperture size, which is regulated by JIS Z-8801-1:2019, of 1 mm, thereby obtaining a thiourethane resin powder that had passed through the sieve (that is, a powder containing a thiourethane resin).

### [Example 1-1]

### <Degradation of Thiourethane Resin with Tetramethylammonium Hydroxide and Ethanol>

### (Reaction Step)

15.0 g of the thiourethane resin powder obtained in Production Example 1 was weighed and all charged into a 300 mL flask equipped with a cooling tube, ethanol (55.3 g; 1.2 mol) as an alcohol and a 25 mass% tetramethylammonium hydroxide (TMAH) aqueous solution (43.8 g; 0.12 mol) as a quaternary ammonium salt aqueous solution were added thereto, heated and stirred at 40°C (reaction temperature) for 3.00 hours (reaction time), thereby obtaining a reaction mixture containing the polythiol composition (reaction step). The content of water (43.8 x 0.75) in a reaction system (15.0 + 55.3 + 43.8) was 28.8 mass% (43.8 x 0.75/(15.0 + 55.3 + 43.8) x 100).

### (Separation Step)

The reaction mixture obtained in the reaction step was cooled to room temperature, and a solid matter was then removed by filtration. Toluene (45.0 g) as a separation solvent was added to the obtained filtrate. The obtained liquid was washed twice with 100 mL of 1M hydrochloric acid to remove excess tetramethylammonium hydroxide (TMAH), then washed twice with 100 mL of water to remove excess hydrochloric acid. A 28 mass% sodium methoxide methanol solution (16.4 g; 0.085 mol) was added to the obtained liquid and stirred. 200.0 g of water was added thereto to extract the soluble component, the generated aqueous extract was washed twice with 45.0 g of toluene, and 21 g of 1M hydrochloric acid was then added thereto and stirred. The soluble component was extracted from the obtained aqueous liquid with 200.0 g of toluene, and a washing and separation operation was performed on the generated extract twice with 100 mL of water, thereby obtaining a toluene solution of the polythiol composition.

A highly polar by-product was removed from the obtained toluene solution with a silica gel column, and toluene was then distilled with a rotary evaporator. On the obtained mixture, removal of a low-boiling component with a vacuum pump and filtration with a 1 micron PTFE membrane filter were performed in this order, thereby obtaining 3.1 g (yield: 41 mass%) of a polythiol composition containing, as main components, 4,8-dimercaptomethyl-1,11-dimercapto-3,6,9-trithiaunane, 4,7-dimercaptomethyl-1,11-dimercapto-3,6,9-trithiaunane, and 5,7-dimercaptomethyl-1,11-dimercapto-3,6,9-trithiaundecane (that is, polythiol components) (separation step).

### [Example 1-2]

### <Degradation of Thiourethane Resin with Benzylamine (Primary Amine) and Ethanol>

### (Reaction Step)

15.0 g of the thiourethane resin powder obtained in Production Example 1 was weighed and all charged into a 300 mL flask equipped with a cooling tube, benzylamine (12.9 g; 0.12 mol) as an amine compound, ethanol (55.3 g; 0.12 mol) as an alcohol, and pure water (18.0 g) were added thereto, heated and stirred at 80°C (reaction temperature) for 2.75 hours (reaction time), thereby obtaining a reaction mixture containing the polythiol composition (reaction step).
The content of water (18.0) in a reaction system (15.0 + 55.3 + 12.9 + 18.0) was 17.8 mass% (18.0/(15.0 + 55.3 + 12.9 + 18.0) x 100).

### (Separation Step)

The reaction mixture obtained in the reaction step was cooled to room temperature, and a solid matter was then removed by filtration. Toluene (50.0 g) as a separation solvent was added to the obtained filtrate. The obtained liquid was washed twice with 100 mL of 1M hydrochloric acid to remove excess benzylamine, then washed twice with 100 mL of water to remove excess hydrochloric acid. A highly polar by-product was removed from the obtained toluene solution with a silica gel column, and toluene was then distilled with a rotary evaporator. On the obtained mixture, removal of a low-boiling component with a vacuum pump and filtration with a 1 micron PTFE membrane filter were performed in this order, thereby obtaining 3.2 g (yield: 42 mass%) of a polythiol composition containing, as main components, 4,8-dimercaptomethyl-1,11-dimercapto-3,6,9-trithiaunane, 4,7-dimercaptomethyl-1,11-dimercapto-3,6,9-trithiaunane, and 5,7-dimercaptomethyl-1,11-dimercapto-3,6,9-trithiaundecane (that is, polythiol components) (separation step).

### [Example 1-3]

### <Degradation of Thiourethane Resin with Methylethanolamine (Secondary Amine) and Ethanol>

A reaction step and a separation step were performed in the same manner as in Example 1-2 except that in Example 1-2, methylethanolamine (9.0 g; 0.12 mol) as a secondary amine and 18.0 g of pure water were used instead of using benzylamine (12.9 g; 0.12 mol) as a primary amine and the reaction time was set to 3.25 hours in the reaction step. Here, what was removed by performing washing twice with 100 mL of 1M hydrochloric acid in the separation step was not excess benzylamine, but excess secondary amine. As a result, 2.9 g (yield: 38 mass%) of a polythiol composition was obtained. The content of water (18.0) in a reaction system (15.0 + 55.3 + 9.0 + 18.0) was 18.5 mass% (18.0/(15.0 + 55.3 + 9.0 + 18.0) x 100).

### [Example 1-4]

### <Degradation of Thiourethane Resin with N,N-dimethylethanolamine (Tertiary Amine) and Ethanol>

A reaction step and a separation step were performed in the same manner as in Example 1-1 except that in Example 1-1, N,N-dimethylethanolamine (10.7 g; 0.12 mol) as a tertiary amine and 18.0 g of pure water were used instead of using the 25 mass% tetramethylammonium hydroxide (TMAH) aqueous solution (43.8 g; 0.12 mol) as a quaternary ammonium salt aqueous solution and the reaction time was set to 3.50 hours in the reaction step. Here, what was removed by performing washing twice with 100 mL of 1M hydrochloric acid in the separation step was not excess tetramethylammonium hydroxide (TMAH), but excess tertiary amine. As a result, 2.4 g (yield: 32 mass%) of a polythiol composition was obtained. The content of water (32.9) in a reaction system (15.0 + 55.3 + 10.7 + 32.9) was 18.2 mass% (18.0/(15.0 + 55.3 + 10.7 + 18.0) x 100).

### [Example 1-5]

### <Degradation of Thiourethane Resin with Diazabicyclo Undecene (DBU) and Ethanol>

A reaction step and a separation step were performed in the same manner as in Example 1-1 except that in Example 1-1, diazabicyclo undecene (DBU) (18.3 g; 0.12 mol) as a cyclic amine and 18.0 g of pure water were used instead of using the 25 mass% tetramethylammonium hydroxide (TMAH) aqueous solution (43.8 g; 0.12 mol) as a quaternary ammonium salt aqueous solution and the reaction time was set to 3.50 hours in the reaction step. Here, what was removed by performing washing twice with 100 mL of 1M hydrochloric acid in the separation step was not excess tetramethylammonium hydroxide (TMAH), but excess diazabicyclo undecene (DBU). As a result, 1.3 g (yield: 17 mass%) of a polythiol composition was obtained. The content of water (18.0) in a reaction system (15.0 + 55.3 + 18.3 + 18.0) was 16.8 mass% (18.0/(15.0 + 55.3 + 18.3 + 18.0) x 100).

### [Example 2]

### <Degradation of Thiourethane Resin with Monoethanolamine>

### (Reaction Step)

10.2 g of the thiourethane resin powder obtained in Production Example 1 was weighed and all charged into a 300 mL flask equipped with a cooling tube, an amine compound, monoethanolamine (5.0 g; 0.081 mol) as an alcohol, toluene (120.0 g) as a reaction solvent, and pure water (12.0 g) were added thereto, heated and stirred at 100°C (reaction temperature) for 2.75 hours (reaction time), thereby obtaining a reaction mixture containing the polythiol composition (reaction step). The content of water (12.0) in a reaction system (10.2 + 5.0 + 120.0 + 12.0) was 8.2 mass% (12.0/(10.2 + 5.0 + 120.0 + 12.0) x 100).

### (Separation Step)

The reaction mixture obtained in the reaction step was cooled to room temperature, and a solid matter was then removed by filtration. The obtained filtrate was washed twice with 50 mL of 1M hydrochloric acid to remove excess monoethanolamine, then washed twice with 100 mL of water to remove excess hydrochloric acid. A highly polar by-product was removed from the obtained toluene solution with a silica gel column, and toluene was then distilled with a rotary evaporator. On the obtained mixture, removal of a low-boiling component with a vacuum pump and filtration with a 1 micron PTFE membrane filter were performed in this order, thereby obtaining 2.6 g (yield: 52 mass%) of a polythiol composition containing, as main components, 4,8-dimercaptomethyl-1,11-dimercapto-3,6,9-trithiaunane, 4,7-dimercaptomethyl-1,11-dimercapto-3,6,9-trithiaunane, and 5,7-dimercaptomethyl-1,11-dimercapto-3,6,9-trithiaundecane (that is, polythiol components) (separation step).

### [Example 3-1]

### <Degradation of Thiourethane Resin with Tetramethylammonium Hydroxide and Methanol>

A reaction step and a separation step were performed in the same manner as in Example 1-1 except that in Example 1-1, methanol (38.4 g; 1.2 mol) was used instead of using ethanol (55.3 g; 1.2 mol), the reaction time was set to 2.75 hours, and the reaction temperature was set to 60°C in the reaction step. As a result, 3.9 g (yield: 51 mass%) of a polythiol composition was obtained. The content of water (43.8 x 0.75) in a reaction system (15.0 + 38.4 + 43.8) was 33.8 mass% (43.8 x 0.75/(15.0 + 38.4 + 43.8) x 100).

### [Example 3-2]

### <Degradation of Thiourethane Resin with Benzylamine (Primary Amine) and Methanol>

A reaction step and a separation step were performed in the same manner as in Example 1-2 except that in Example 1-2, methanol (38.4 g; 1.2 mol) was used instead of using ethanol (55.3 g; 1.2 mol), the reaction time was set to 2.50 hours, and the reaction temperature was set to 60°C in the reaction step. As a result, 3.6 g (yield: 48 mass%) of a polythiol composition was obtained. The content of water (18.0) in a reaction system (15.0 + 38.4 + 12.9 + 18.0) was 21.4 mass% (18.0/(15.0 + 38.4 + 12.9 + 18.0) x 100).

### [Example 3-3]

### <Degradation of Thiourethane Resin with Methylethanolamine (Secondary Amine) and Methanol>

A reaction step and a separation step were performed in the same manner as in Example 1-3 except that in Example 1-3, methanol (38.4 g; 1.2 mol) was used instead of using ethanol (55.3 g; 1.2 mol), the reaction time was set to 3.00 hours, and the reaction temperature was set to 60°C in the reaction step. As a result, 3.1 g (yield: 41 mass%) of a polythiol composition was obtained. The content of water (18.0) in a reaction system (15.0 + 38.4 + 9.0 + 18.0) was 23.0 mass% (18.0/(15.0 + 38.4 + 9.0 + 18.0) x 100).

### [Example 3-4]

### <Degradation of Thiourethane Resin with N,N-dimethylethanolamine (Tertiary Amine) and Methanol>

A reaction step and a separation step were performed in the same manner as in Example 1-4 except that in Example 1-4, methanol (38.4 g; 1.2 mol) was used instead of using ethanol (55.3 g; 1.2 mol), the reaction time was set to 3.00 hours, and the reaction temperature was set to 60°C in the reaction step. As a result, 2.9 g (yield: 38 mass%) of a polythiol composition was obtained. The content of water (18.0) in a reaction system (15.0 + 38.4 + 10.7 + 18.0) was 21.9 mass% (18.0/(15.0 + 38.4 + 10.7 + 18.0) x 100).

### [Example 3-5]

### <Degradation of Thiourethane Resin with Diazabicyclo Undecene (DBU) and Methanol>

A reaction step and a separation step were performed in the same manner as in Example 1-5 except that in Example 1-5, methanol (38.4 g; 1.2 mol) was used instead of using ethanol (55.3 g; 1.2 mol), the reaction time was set to 3.25 hours, and the reaction temperature was set to 60°C in the reaction step. As a result, 1.6 g (yield: 21 mass%) of a polythiol composition was obtained. The content of water (18.0) in a reaction system (15.0 + 38.4 + 18.3 + 18.0) was 20.0 mass% (18.0/(15.0 + 38.4 + 18.3 + 18.0) x 100).

### [Example 4-1]

### <Degradation of Thiourethane Resin with Tetramethylammonium Hydroxide and Isopropanol>

A reaction step and a separation step were performed in the same manner as in Example 1-1 except that in Example 1-1, isopropanol (2-propanol) (72.1 g; 1.2 mol) was used instead of using ethanol (55.3 g; 1.2 mol) in the reaction step. As a result, 2.7 g (yield: 36 mass%) of a polythiol composition was obtained. The content of water (43.8 x 0.75) in a reaction system (15.0 + 72.1 + 43.8) was 25.1 mass% (43.8 x 0.75/(15.0 + 72.1 + 43.8) x 100).

### [Example 4-2]

### <Degradation of Thiourethane Resin with Benzylamine (Primary Amine) and Isopropanol>

A reaction step and a separation step were performed in the same manner as in Example 1-2 except that in Example 1-2, isopropanol (2-propanol) (72.1 g; 1.2 mol) and pure water (72.0 g) were used instead of using ethanol (55.3 g; 1.2 mol) and pure water (18.0 g) and the reaction temperature was set to 100°C in the reaction step. As a result, 3.4 g (yield: 45 mass%) of a polythiol composition was obtained. The content of water (72.0) in a reaction system (15.0 + 72.1 + 12.9 + 72.0) was 41.9 mass% (72.0/(15.0 + 72.1 + 12.9 + 72.0) x 100).

### [Example 4-3]

### <Degradation of Thiourethane Resin with Methylethanolamine (Secondary Amine) and Isopropanol>

A reaction step and a separation step were performed in the same manner as in Example 1-3 except that in Example 1-3, isopropanol (2-propanol) (72.1 g; 1.2 mol) and pure water (90.0 g) were used instead of using ethanol (55.3 g; 1.2 mol) and pure water (18.0 g) and the reaction temperature was set to 100°C in the reaction step. As a result, 2.2 g (yield: 29 mass%) of a polythiol composition was obtained. The content of water (90.0) in a reaction system (15.0 + 72.1 + 9.0 + 90.0) was 48.4 mass% (90.0/(15.0 + 72.1 + 9.0 + 90.0) x 100).

### [Example 4-4]

### <Degradation of Thiourethane Resin with N,N-dimethylethanolamine (Tertiary Amine) and Isopropanol>

A reaction step and a separation step were performed in the same manner as in Example 1-4 except that in Example 1-4, isopropanol (2-propanol) (72.1 g; 1.2 mol) and pure water (54.0 g) were used instead of using ethanol (55.3 g; 1.2 mol) and pure water (18.0 g), the reaction time was set to 3.25 hours, and the reaction temperature was set to 100°C in the reaction step. As a result, 2.7 g (yield: 36 mass%) of a polythiol composition was obtained. The content of water (54.0) in a reaction system (15.0 + 72.1 + 10.7 + 32.9) was 35.6 mass% (54.0/(15.0 + 72.1 + 10.7 + 54.0) x 100).

### [Example 4-5]

### <Degradation of Thiourethane Resin with Diazabicyclo Undecene (DBU) and Isopropanol>

A reaction step and a separation step were performed in the same manner as in Example 1-5 except that in Example 1-5, isopropanol (2-propanol) (72.1 g; 1.2 mol) was used instead of using ethanol (55.3 g; 1.2 mol) and the reaction temperature was set to 100°C in the reaction step. As a result, 1.4 g (yield: 19 mass%) of a polythiol composition was obtained. The content of water (18.0) in a reaction system (15.0 + 72.1 + 18.3 + 18.0) was 14.6 mass% (18.0/(15.0 + 72.1 + 18.3 + 18.0) x 100).

### [Example 5-1]

### <Degradation of Thiourethane Resin with Tetramethylammonium Hydroxide and Benzyl Alcohol>

A reaction step and a separation step were performed in the same manner as in Example 1-1 except that in Example 1-1, benzyl alcohol (129.8 g; 1.2 mol) was used instead of using ethanol (55.3 g; 1.2 mol) and the reaction time was set to 2.75 hours in the reaction step. As a result, 3.0 g (yield: 40 mass%) of a polythiol composition was obtained. The content of water (43.8 x 0.75) in a reaction system (15.0 + 129.8 + 43.8) was 17.4 mass% (43.8 x 0.75/(15.0 + 129.8 + 43.8) x 100).

### [Example 5-2]

### <Degradation of Thiourethane Resin with Benzylamine (Primary Amine) and Benzyl Alcohol>

A reaction step and a separation step were performed in the same manner as in Example 1-2 except that in Example 1-2, benzyl alcohol (129.8 g; 1.2 mol) was used instead of using ethanol (55.3 g; 1.2 mol) and the reaction temperature was set to 100°C in the reaction step. As a result, 3.1 g (yield: 41 mass%) of a polythiol composition was obtained. The content of water (18.0) in a reaction system (15.0 + 129.8 + 12.9 + 18.0) was 10.2 mass% (18.0/(15.0 + 129.8 + 12.9 + 18.0) x 100).

### [Example 5-3]

### <Degradation of Thiourethane Resin with Methylethanolamine (Secondary Amine) and Benzyl Alcohol>

A reaction step and a separation step were performed in the same manner as in Example 1-3 except that in Example 1-3, benzyl alcohol (129.8 g; 1.2 mol) was used instead of using ethanol (55.3 g; 1.2 mol), the reaction time was set to 3.00 hours, and the reaction temperature was set to 100°C in the reaction step. As a result, 2.7 g (yield: 36 mass%) of a polythiol composition was obtained. The content of water (18.0) in a reaction system (15.0 + 129.8 + 12.9 + 32.9) was 10.5 mass% (18.0/(15.0 + 129.8 + 9.0 + 18.0) x 100).

### [Example 5-4]

### <Degradation of Thiourethane Resin with N,N-dimethylethanolamine (Tertiary Amine) and Benzyl Alcohol>

A reaction step and a separation step were performed in the same manner as in Example 1-4 except that in Example 1-4, benzyl alcohol (129.8 g; 1.2 mol) was used instead of using ethanol (55.3 g; 1.2 mol), the reaction time was set to 3.25 hours, and the reaction temperature was set to 100°C in the reaction step. As a result, 2.3 g (yield: 30 mass%) of a polythiol composition was obtained. The content of water (18.0) in a reaction system (15.0 + 129.8 + 10.7 + 18.0) was 10.4 mass% (18.0/(15.0 + 129.8 + 10.7 + 18.0) x 100).

### [Example 5-5]

### <Degradation of Thiourethane Resin with Diazabicycloundecene (DBU) and Benzyl Alcohol>

A reaction step and a separation step were performed in the same manner as in Example 5-5 except that in Example 5-5, benzyl alcohol (129.8 g; 1.2 mol) was used instead of using ethanol (55.3 g; 1.2 mol), the reaction time was set to 3.50 hours, and the reaction temperature was set to 100°C in the reaction step. As a result, 1.8 g (yield: 24 mass%) of a polythiol composition was obtained. The content of water (18.0) in a reaction system (15.0 + 129.8 + 18.3 + 18.0) was 9.9 mass% (18.0/(15.0 + 129.8 + 18.3 + 18.0) x 100).

### [Comparative Example 1]

### <Degradation of Thiourethane Resin with Monoethanolamine>

### (Reaction Step)

10.2 g of the thiourethane resin powder obtained in Production Example 1 was weighed and all charged into a 300 mL 300 mL flask equipped with a cooling tube, an amine compound, monoethanolamine (5.0 g; 0.081 mol) as an alcohol, and toluene (120.0 g) as a reaction solvent were added thereto, heated and stirred at 100°C (reaction temperature) for 6.00 hours (reaction time), thereby obtaining a reaction mixture containing the polythiol composition (reaction step). The content of water in a reaction system was 0.0 mass%.

### (Separation Step)

The reaction mixture obtained in the reaction step was cooled to room temperature, and a solid matter was then removed by filtration. The obtained filtrate was washed twice with 50 mL of 1M hydrochloric acid to remove excess monoethanolamine, then washed twice with 50 mL of water to remove excess hydrochloric acid. A highly polar by-product was removed from the obtained toluene solution with a silica gel column, and toluene was then distilled with a rotary evaporator. On the obtained mixture, removal of a low-boiling component with a vacuum pump and filtration with a 1 micron PTFE membrane filter were performed in this order, thereby obtaining 2.8 g (yield: 54 mass%) of a polythiol composition containing, as main components, 4,8-dimercaptomethyl-1,11-dimercapto-3,6,9-trithiaunane, 4,7-dimercaptomethyl-1,11-dimercapto-3,6,9-trithiaunane, and 5,7-dimercaptomethyl-1,11-dimercapto-3,6,9-trithiaundecane (that is, polythiol components) (separation step).

### [Comparative Example 2-1]

### <Degradation of Thiourethane Resin with Tetramethylammonium Hydroxide and Methanol>

A reaction step and a separation step were performed in the same manner as in Example 3-1 except that in Example 3-1, a 10 mass% tetramethylammonium hydroxide (TMAH) methanol solution (109.4 g; 0.12 mol) was used instead of using methanol (38.4 g; 1.2 mol) and the 25 mass% tetramethylammonium hydroxide (TMAH) aqueous solution (43.8 g; 0.12 mol), the reaction time was set to 6.00 hours, and the reaction temperature was set to 60°C in the reaction step. As a result, 2.9 g (yield: 38 mass%) of a polythiol composition was obtained. The content of water in a reaction system was 0.0 mass%.

### [Comparative Example 2-2]

### <Degradation of Thiourethane Resin with Benzylamine (Primary Amine) and Methanol>

A reaction step and a separation step were performed in the same manner as in Example 3-2 except that in Example 3-2, the reaction time was set to 5.75 hours and 18.0 g of pure water was not used in the reaction step. As a result, 2.5 g (yield: 33 mass%) of a polythiol composition was obtained. The content of water in a reaction system was 0.0 mass%.

### [Comparative Example 2-3]

### <Degradation of Thiourethane Resin with Methylethanolamine (Secondary Amine) and Methanol>

A reaction step and a separation step were performed in the same manner as in Example 3-3 except that in Example 3-3, the reaction time was set to 6.50 hours and 18.0 g of pure water was not used in the reaction step. As a result, 2.1 g (yield: 28 mass%) of a polythiol composition was obtained. The content of water in a reaction system was 0.0 mass%.

### [Comparative Example 2-4]

### <Degradation of Thiourethane Resin with N,N-dimethylethanolamine (Tertiary Amine) and Methanol>

A reaction step and a separation step were performed in the same manner as in Example 3-4 except that in Example 3-4, the reaction time was set to 6.25 hours and 32.9 g of pure water was not used in the reaction step. As a result, 1.8 g (yield: 24 mass%) of a polythiol composition was obtained. The content of water in a reaction system was 0.0 mass%.

### [Comparative Example 2-5]

### <Degradation of Thiourethane Resin with Diazabicyclo Undecene (DBU) and Methanol>

A reaction step and a separation step were performed in the same manner as in Example 3-5 except that in Example 3-5, the reaction time was set to 6.50 hours and 32.9 g of pure water was not used in the reaction step. As a result, 1.1 g (yield: 15 mass%) of a polythiol composition was obtained. The content of water in a reaction system was 0.0 mass%.

### [Evaluation of Yield]

The yields of Examples 1-1 to 5-5 and Comparative Examples 1 to 2-5 are shown in Table 1.

### [Definition of Reaction Time]

The progress of each reaction was tracked by silica gel TLC (thin layer chromatography) every 15 minutes from the start of the reaction. The time taken until the reaction was saturated was visually confirmed and recorded as the reaction saturation time, and the reaction saturation time was regarded as the reaction time. The results are shown in Table 1. Regarding the "reaction saturation time" mentioned herein, in the present specification, a time when a spot near an Rf value of 0.7 is visually confirmed for a small amount of a sample that has undergone a post treatment of changing thiolate to thiol by TLC (developing solvent; ethyl acetate:toluene = 1:10 to 1:7, detection reagent; phosphomolybdic acid ethanol solution) and no further progression is observed is defined as the reaction termination time, that is, the reaction saturation time. Here, the Rf value is a value obtained by dividing the movement distance of the compound by the movement distance of the solvent on the TLC plate. In addition, the phosphomolybdic acid ethanol reagent is a solution adjusted by dissolving 5 g of sodium phosphomolybdate n hydrate in 100 mL of ethanol.

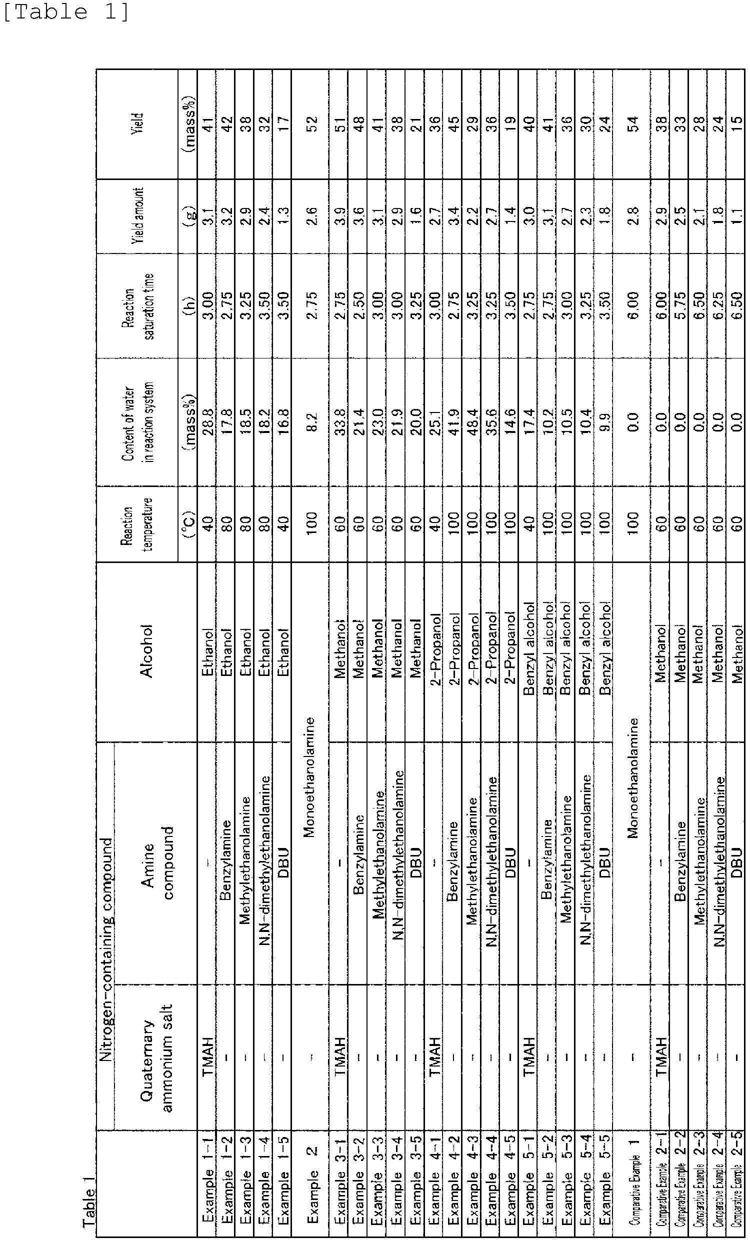

It is found from Table 1 that according to the methods for producing a polythiol composition of Examples 1-1 to 5-5, which include the reaction step of reacting the thiourethane resin and the nitrogen-containing compound in the presence of water to generate a polythiol composition, polythiol compositions can be produced using a thiourethane resin as a starting raw material in a short reaction time.

The embodiment disclosed here is only illustrative in all respects and should not be considered as restrictive. The scope of the present disclosure is indicated by the scope of the claims rather than the above description, and is intended to include meanings equivalent to the scope of the claims and all modifications within the scope of the claims.

In the present disclosure, regarding examples, contents, and various physical properties of each of the above components, matters described in the detailed description of the invention as illustrations or preferable ranges may be optionally combined together.

In addition, as long as the compositions described in the examples can be adjusted to the compositions described in the detailed description of the invention, it is possible to perform the invention in the same manner as the examples throughout the entire claimed composition scopes.

## Claims

1. A method for producing a polythiol composition comprising:
a reaction step of reacting a thiourethane resin and a nitrogen-containing compound that is at least one selected from the group consisting of a quaternary ammonium salt and an amine compound in the presence of water to generate a polythiol composition.

2. The method for producing the polythiol composition according to claim 1, wherein a content of water in a reaction system of the reaction step is 2 to 50 mass%.

3. The method for producing the polythiol composition according to claim 1 or 2, wherein the quaternary ammonium salt comprises a quaternary ammonium cation and a counter anion, and the counter anion is a hydroxide ion.

4. The method for producing the polythiol composition according to any one of claims 1 to 3, wherein the amine compound is at least one selected from the group consisting of a primary amine, a secondary amine, and a tertiary amine.

5. The method for producing the polythiol composition according to any one of claims 1 to 4, wherein the reaction system in the reaction step further contains an alcohol.

6. The method for producing a polythiol composition according to claim 5, wherein the alcohol contains one or more alcohols miscible with water.

7. A method for producing a polymerizable composition, comprising:
a step of producing a polythiol composition by the method for producing a polythiol composition according to any one of claims 1 to 6; and
a step of mixing a polythiol composition containing the produced polythiol composition and a polyisocyanate compound to obtain a polymerizable composition containing the polythiol composition and the polyisocyanate compound.

8. A method for producing a resin, comprising:
a step of producing a polymerizable composition by the method for producing a polymerizable composition according to claim 7; and
a step of curing the polymerizable composition to obtain a resin.
